# EUROPEAN PATENT SPECIFICATION

(11) **EP 2 964 740 B1**
(45) Date of publication and mention of the grant of the patent: **04.10.2017**
(21) Application number: 14715151.8
(22) Date of filing: 05.03.2014
(51) Int. Cl.: C11D 1/66, C11D 1/825, C11D 1/52, C11D 3/32, A61K 8/42, A61K 31/16, A61Q 5/00, A61Q 5/02, A61Q 5/12, A61Q 9/02, A61Q 9/04, A61Q 13/00, A61Q 15/00, A61Q 19/00, A61Q 19/10, A61Q 90/00

(54) **MIXED SUGAR-BASED AMIDE SURFACTANT COMPOSITIONS**
ZUSAMMENSETZUNGEN AUS GEMISCHTEN ZUCKERAMIDBASIERTEN TENSIDEN
COMPOSITIONS COMPRENAT DES COMBINAISONS DE TENSIO-ACTIFS DÉRIVÉS D'AMIDES DE SUCRES

(30) Priority: 05.03.2013 US 201361772736 P
(43) Date of publication of application: 13.01.2016
(73) Proprietor: The Procter & Gamble Company, Cincinnati, OH 45202 (US)
(72) Inventor: SCHEIBEL, Jeffrey, John, Cincinnati, Ohio 45202 (US); WEST, Ryan, Michael, Cincinnati, Ohio 45202 (US); CRON, Scott, Leroy, Cincinnati, Ohio 45202 (US); VINSON, Phillip, Kyle, Cincinnati, Ohio 45202 (US); STEFFEY, Melinda, Phyllis, Cincinnati, Ohio 45202 (US); DELPLANCKE, Patrick, Firmin August, Cincinnati, Ohio 45202 (US); REILMAN, Randall, Thomas, Cincinnati, Ohio 45202 (US)
(74) Representative: Siddiquee, Sanaul Kabir
(86) International application number: PCT/US2014/020459
(87) International publication number: WO 2014/138141

(56) References cited:
- EP-A1- 0 550 557
- US-A- 5 750 733
- MARIA ALEXEEVA ET AL: "Micellar solution-solid phase equilibrium for ternary systems containing two N-alkanoyl-N-methylglucamine homologues", FLUID PHASE EQUILIBRIA, vol. 136, no. 1-2, 1 November 1997 (1997-11-01), pages 173-183, XP055127343, ISSN: 0378-3812, DOI: 10.1016/S0378-3812(97)00119-2

## Description

### FIELD OF THE INVENTION

Novel mixtures of sugar amides or sugar amines are disclosed that have improved thermal properties over the individual components.

### BACKGROUND OF THE INVENTION

Surfactants are the single most important cleaning ingredient in cleaning products. Environmental regulations, consumer habits, and consumer practices have forced new developments in the surfactant industry to produce lower-cost, higher-performing and environmentally friendly products. Examples of developments in the surfactant industry are described by J. Scheibel in the Journal of Surfactants and Detergents, "The Evolution of Anionic Surfactant Technology to Meet the Requirements of the Laundry Detergent Industry," volume 7, number 4, October, 2004 ("Scheibel JSD Article" hereinafter). Today, challenges facing the surfactant industry include colder wash temperatures, less efficient builders, liquid products without calcium control, and a push for reduced surfactant use overall because of the perceived environmental impact of surfactants.

Sugar amide surfactants were commercialized and used by Procter & Gamble for a number of years in various consumer products. See Chemical Economics Handbook Marketing Research Report, Janshekar et al, "Surfactants, Household Detergents and Their Raw Materials", June 2010, 43. They were based on existing feedstocks available at that time such as coconut and palm kernel oils and glucose as the sugar source for the n-methylglucamine. The physical properties of pure sugar amides can be found in literature references; see Alexeeva et al, Fluid Phase Equilibria 136 (1997) pp 173-183 (Elsevier edit.); see Zhu et al, Journal of Surfactants and Detergents, Vol. 2, No. 3, July 1999, pp 357 - 362, ("Zhu et al" hereinafter). Previously commercialized sugar amide surfactants based solely on glucose and the methyl esters of coconut and palm kernel oils suffered from high thermal properties such as melting point and Krafft point, which limited the true potential of the surfactant for broader application in consumer products. See also Laughlin et al, Surfactant Science Series (1998), 74 (Novel Surfactants), 1-30 and Laughlin et al, Surfactant Science Series (2003), second ed., 114 (Novel Surfactants), Chapter 1, 1-36. Reference may also be made to US 5,750,733 (Lever Brothers Cy, Division of Conopco Inc). It has now been discovered that new feedstocks based on both the surfactant tail as well as the sugar head group allow for improved physical properties of sugar amide surfactant mixtures and thus improved formulatability. New cellulosic sugar mixtures can give novel and improved sugar amines and sugar amide surfactants with improved thermal properties. Furthermore new sources of unique methyl esters from both bioengineering and or co-metathesis of fats and oils can also provide novel and improved sugar amide surfactant mixtures.

Furthermore, there is a desire by consumers for sustainable products and ingredients. The current existing sustainable surfactants have limitations in terms of formulatability, cost and formulation flexibility. Thus there is a need for high performing sustainable surfactants in the marketplace with improved properties over the existing materials.

### SUMMARY OF THE INVENTION

The present invention provides a novel mixture of sugar amides that have improved thermal properties over the individual components. The mixture comprising a first chemical and a second chemical, wherein said first chemical has the chemical structure of Formula I and said second chemical has the chemical structure of Formula II: 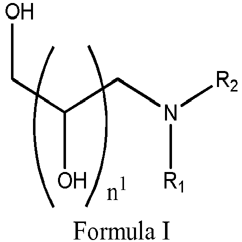 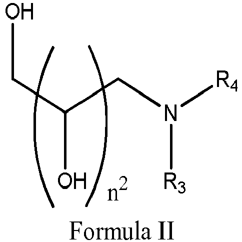 wherein n¹ is 2 to 4; n² is 1 to 3; n¹ is greater than n²; R₁ and R₃ are independently selected from C₁-C₁₆ alkyl, C₁-C₃ hydroxy- or methoxy-alkyl and; R₂ and R₄ are independently selected from a structure of Formula III 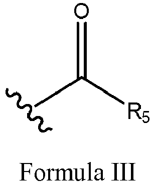 wherein: R₅ is C₇-C₂₃ alkyl, mono-alkenyl, di-alkenyl, tri-alkenyl, hydroxy-alkyl, or hydroxyalkenyl;
and mixtures thereof.

According to one embodiment, n¹ is equal to 4. The chemical fragment in Formula I, not including the nitrogen atom R₁ or R₂, has 6 carbon and 5 oxygen atoms. This chemical fragment can be derived from a six carbon sugar including but not limited to glucose, mannose or galactose.

According to a second embodiment, n² is equal to 3. The chemical fragment in Formula II not including the nitrogen atom, R₃ or R₄ has 5 carbon, and 4 oxygen atoms. This chemical fragment can be derived from a five carbon sugar including but not limited to xylose or arabinose.

According to another embodiment, n¹ is equal to 4 and n² is equal to 3. The chemical fragment in Formula I not including the nitrogen atom, R₁ or R₂ has 6 carbon and 5 oxygen atoms. This chemical fragment can be derived from a six carbon sugar including but not limited to glucose, mannose or galactose. The chemical fragment in Formula II not including the nitrogen atom, R₃ or R₄ has 5 carbon, and 4 oxygen atoms. This chemical fragment can be derived from a five carbon sugar including but not limited to xylose or arabinose.

According to another embodiment, n¹ is equal to 3. The chemical fragment in Formula I not including the nitrogen atom, R₁ or R₂ has 5 carbon and 4 oxygen atoms. This chemical fragment can be derived from a five carbon sugar including but not limited to xylose or arabinose.

According to another embodiment, n² is equal to 1. The chemical fragment in Formula II not including the nitrogen atom, R₃ or R₄ has 3 carbon, and 2 oxygen atoms. This chemical fragment can be derived from a three carbon sugar or polyol including but not limited to glyceraldehyde or glycerol.

### BRIEF DESCRIPTION OF THE DRAWINGS

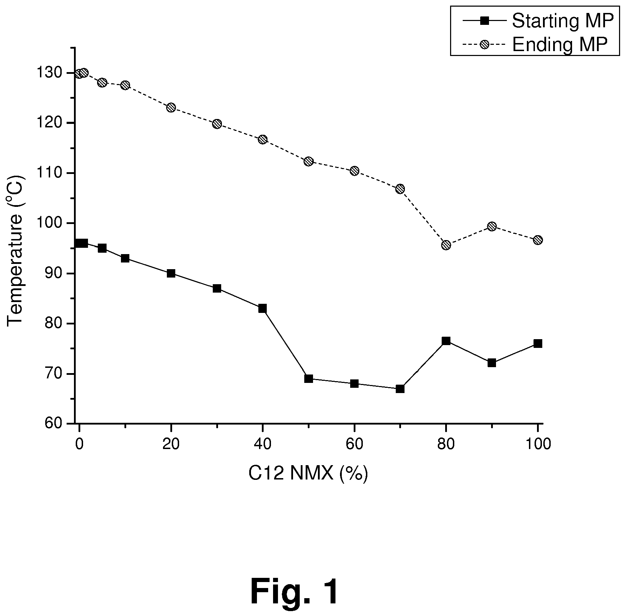

Fig. 1 is a graphic display of the data from Table 1 regarding the visual melting of C12-NMG:C12-NMX mixtures.

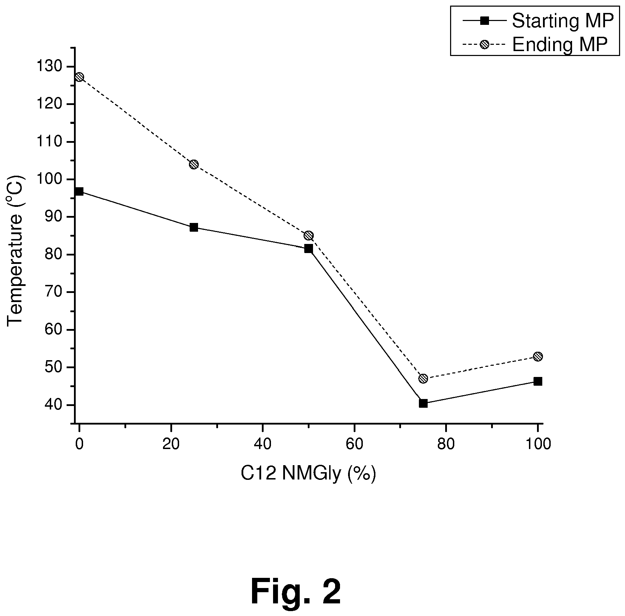

Fig. 2 is a graphic display of the data from Table 2 regarding the visual melting of C12-NMG:C12-NMGly mixtures.

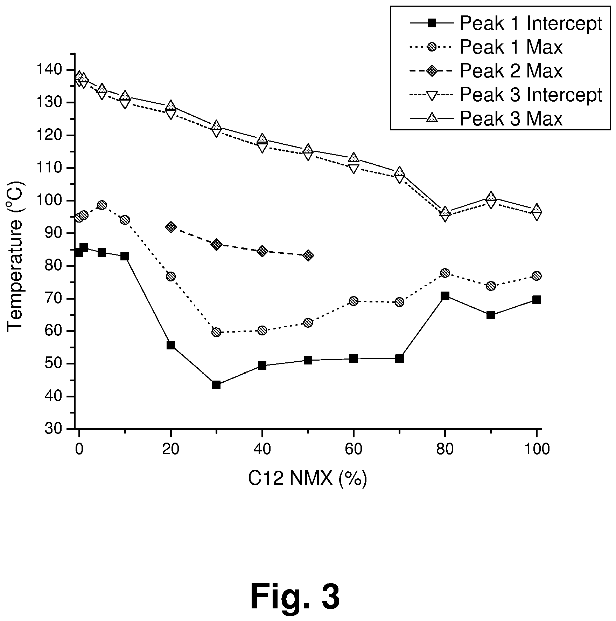

Fig. 3 is a graphic display of the data from Table 3 regarding the thermal transition determination of C12-NMG:C12-NMX by DSC.

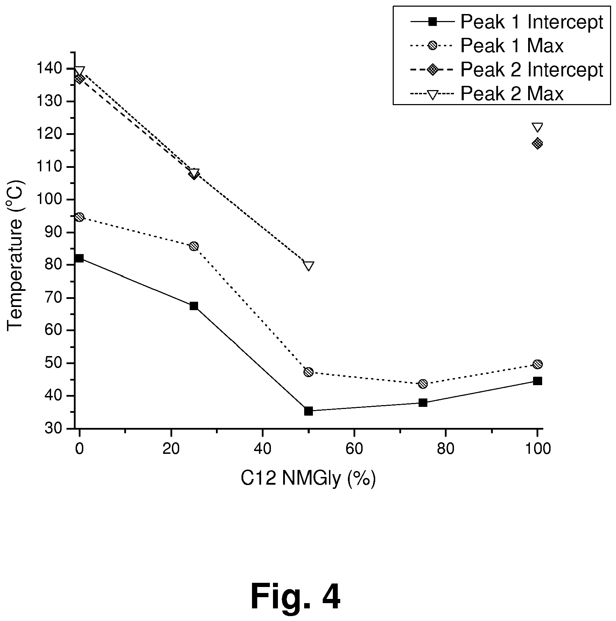

Fig. 4 is a graphic display of the data from Table 4 regarding the thermal transition determination of C12-NMG:C12-NMGly by DSC.

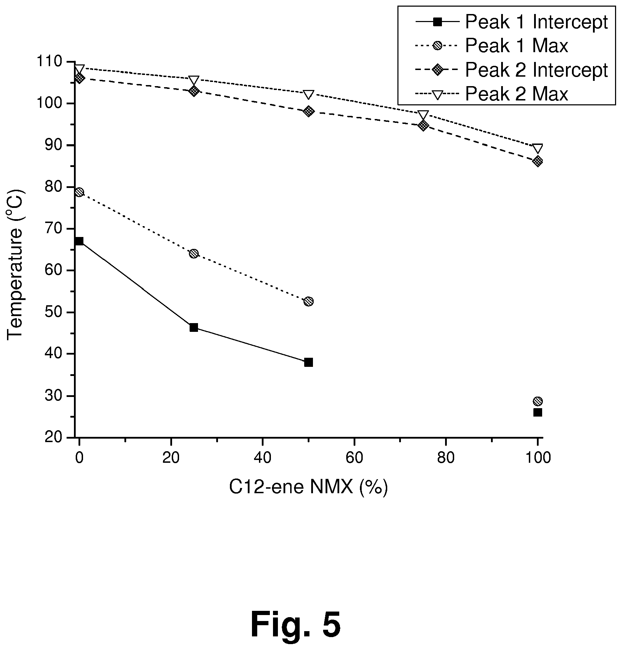

Fig. 5 is a graphic display of the data from Table 5 regarding the thermal transition determination of C12-ene-NMG:C12-ene-NMX by DSC.

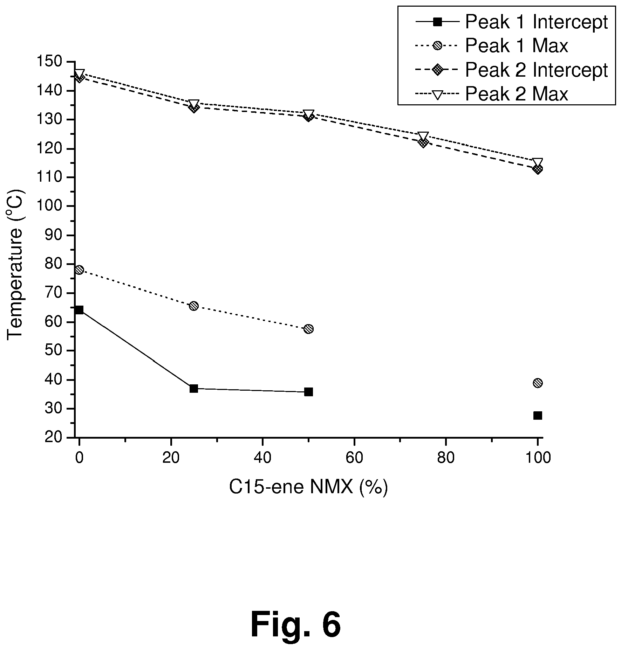

Fig. 6 is a graphic display of the data from Table 6 regarding the thermal transition determination of C15-ene-NMG:C15-ene-NMX by DSC.

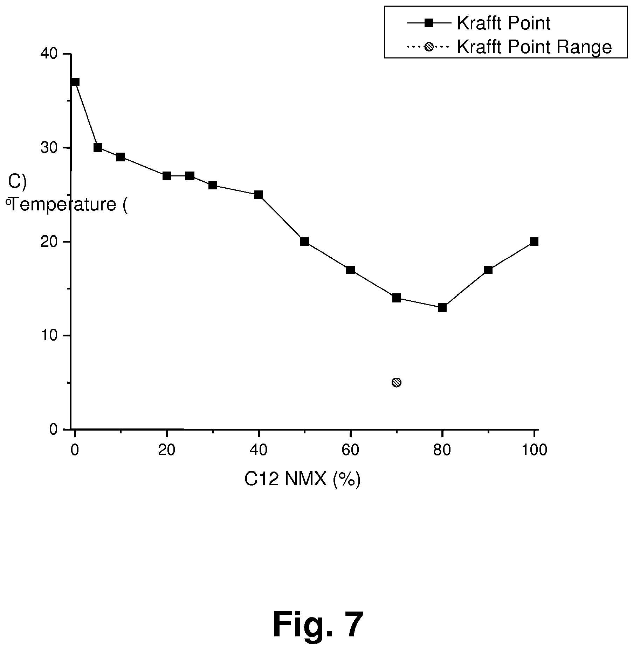

Fig. 7 is a graphic display of the data from Table 7 regarding the Krafft point measurements of 1 wt% mixtures of C12-NMG and C12-NMX.

### DETAILED DESCRIPTION OF THE INVENTION

### Definitions

As used herein "consumer product" means consumer and institutional products, including but not limited to laundry, dishwashing, and hard surface cleaning products, other cleaners, and cleaning systems all for the care and cleaning of inanimate surfaces, as well as fabric conditioner products and other products designed specifically for the care and maintenance of fabrics, and air care products. This definition does not include products (a) intended to be used to clean contact lenses, or ultrafiltration membranes, or (b) in healing wounds or for the medical treatment of skin conditions. Such consumer products are generally intended to be used or consumed in the form in which they are sold.

As used herein, the term "cleaning and/or treatment composition" is a subset of consumer products, Such products include, but are not limited to, products for treating fabrics, hard surfaces and any other surfaces in the area of fabric and home care, including: air care including air fresheners and scent delivery systems, car care, dishwashing, fabric conditioning (including softening and/or freshening), laundry detergency, laundry and rinse additive and/or care, hard surface cleaning and/or treatment including floor and toilet bowl cleaners, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use: car or carpet shampoos, bathroom cleaners including toilet bowl cleaners; as well as cleaning auxiliaries such as bleach additives and "stain-stick" or pre-treat types, substrate-laden products such as dryer added sheets.

As used herein, the term "fabric and/or hard surface cleaning and/or treatment composition" is a subset of cleaning and treatment compositions that includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, car or carpet shampoos, bathroom cleaners including toilet bowl cleaners; fabric conditioning products including softening and/or freshening that may be in liquid, solid and/or dryer sheet form; as well as cleaning auxiliaries such as bleach additives and "stain-stick" or pre-treat types, substrate-laden products such as dryer added sheets. All of such products which are applicable may be in standard, concentrated or even highly concentrated form even to the extent that such products may in certain aspect be non-aqueous.

As used herein, articles such as "a" and "an" when used in a claim, are understood to mean one or more of what is claimed or described.

As used herein, the terms "include", "includes" and "including" are meant to be non-limiting.

As used herein, the term "solid" includes granular, powder, bar and tablet product forms.

As used herein, the term "fluid" includes liquid, gel, paste and gas product forms.

As used herein, the term "situs" includes fabrics, garments, and/or hard surfaces.

As used herein, the terms mono-alkenyl, di-alkenyl and tri-alkenyl refers to the number of double bonds in the single C₇-C₂₃ alkenyl chain represented by R5: mono-alkenyl has one double bond, di-alkenyl has two double bonds, and tri-alkenyl has three double bonds.

As used herein, the term alkyl refers to a monovalent hydrocarbon fragment that can be linear, or branched, with the general formula CₙH₂ₙ₊₁, where n is an integer, or cyclic with the general formula CₙH_{2n+1-2#}, where n is an integer and # is the number of cyclic groups in the hydrocarbon fragment. In cases where a specific length alkyl chain is referenced, the value of n is given before the term alkyl in the subscript; for example Cₙ alkyl.

As used herein, the term hydroxyl-alkyl has the same definition as alkyl with the exception that one hydrogen atom in the hydrocarbon fragment is replaced by a hydroxyl group which contains one oxygen atom and one hydrogen atom.

As used herein, the term methoxy-alkyl has the same definition as alkyl with the exception that one hydrogen atom in the hydrocarbon fragment is replaced by a methoxy group which contains one oxygen atom, one carbon atom and three hydrogen atoms.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated. It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### Sugar Amides and Amines

Sugars suitable for making a mixture of sugar amides or sugar amines can come from a variety of sources. Glucose can be derived from starch, the disaccharide sucrose or cellulose. Xylose can be derived from xylans or from the hemicellulosic portion of a lignocellulosic material. Sugar amides or sugar amines made from these individual sources can then be combined together to provide a mixture of sugar amides or sugar amines.

Alternatively, the sugars can come from a single source such as a lignocellulosic material. Lignocellulosic materials containing both cellulose and hemicellulose can provide both glucose and xylose for reaction into mixed sugar amines and mixed sugar amides. Suitable lignocellulosic materials include wood and wood residues from hardwoods and softwoods; crops, energy crops, agricultural residues and grasses such as wheat, straw, switchgrass, sorgum, bagasse, and stover; or wastes such as industrial or municipal solid waste.

Alternatively, the sugars can come from a combination of sources including a primarily glucose containing source such as starch and a mixed sugar containing source such as a lignocellulosic material for example, wheat.

Other groups besides a fragment derived from sugar are attached to the nitrogen atom. In Formula I and Formula II, R₁ and R₃ are independently selected from hydrogen, C₁-C₁₆ alkyl, C₁-C₃ hydroxy- or methoxy-alkyl while R₂ and R₄ are independently selected from Formula III wherein R₅ is C₇-C₂₃ alkyl, C₇-C₂₃ mono-alkenyl, C₇-C₂₃ di-alkenyl, C₇-C₂₃ tri-alkenyl, C₇-C₂₃ hydroxyl-alkyl, C₇-C₂₃ hydroxyl-alkenyl, and mixtures thereof. In another embodiment of sugar amides, R₅ is C₁₁ alkyl, C₁₃ alkyl, and mixtures thereof.

In one embodiment of sugar amides, R₁ is methyl, while R₂ has the structure of Formula III.

In another embodiment of sugar amides, R₃ is methyl, while R₄ has the structure of Formula III.

In another embodiment of sugar amides, R₁ is methyl, R₂ has the structure of Formula III, R₃ is methyl and R₄ has the structure of Formula III, where Formula III is independently selected for R₂ and R₄.

In another embodiment of sugar amides, R₅ is C₇-C₂₃ alkyl, mono-alkenyl, di-alkenyl, tri-alkenyl, hydroxyl-alkyl, or hydroxyl-alkenyl and mixtures thereof.

In another embodiment of sugar amides, R₅ is C₁₁, C₁₃ alkyl, and mixture thereof.

In one embodiment, the ratio of the first chemical (based on Formula I) to the second chemical (based on Formula II) is from 99.5:0.5 to 0.5:99.5 in the mixture.

In another embodiment, the ratio of the first chemical (based on Formula I) to the second chemical (based on Formula II) is from 95:5 to 5:95 in the mixture.

In another embodiment, the ratio of the first chemical (based on Formula I) to the second chemical (based on Formula II) is from 50:50 to 0.5:99.5 in the mixture.

In another embodiment of sugar amides, the ratio of the first chemical (based on Formula I) to the second chemical (based on Formula II) is from 50:50 to 0.5:99.5 in the mixture, n¹ is equal to 4, n² is equal to 3, R₁ and R₃ are methyl groups, and R₂ and R₄ have the structure of Formula III and R5 is a mixture of C₁₁ and C₁₃ alkyl, wherein the weight ratio of C₁₁ to C₁₃ alkyl is between 99:1 and 60:40. The chemical fragment in Formula I not including the nitrogen atom, R₁ or R₂ has 6 carbon and 5 oxygen atoms. This chemical fragment can be derived from a six carbon sugar including but not limited to glucose, mannose or galactose. The chemical fragment in Formula II not including the nitrogen atom, R₃ or R₄ has 5 carbon, and 4 oxygen atoms. This chemical fragment can be derived from a five carbon sugar or polyol including but not limited to xylose or arabinose and mixtures thereof. Suitable sources of the fatty esters required to make the sugar amide compositions to provide the structure of Formula III can come from any of the following sources: triglycerides, fatty acids, fatty esters, bioengineered fatty acids or esters, or synthetic esters. Suitable fatty acids, or esters of fatty acids can come from the following oils: vegetable oil such as coconut oil, palm kernel oil, camelina oil, cuphea oil, soybean oil, palm oil, and canola oil. Other suitable oils are tung oil, meadowfoam oil, coriander oil, camelina oil, safflower oil, jatropha oil, cramby oil, high erucic rapeseed oil, algal oil, high oleoyl soybean oil (HOSBO), high oleoyl sunflower oil, castor oil, animal fats, and waste fats and oils as non-limiting examples.

Another source of both even and odd chain length, saturated and unsaturated fatty acids or esters for use in the making the amides of the invention can come from co-metathesis of short chain olefins with the various oils described above. U.S. Patent Application Publication No. 2006/0079704, discloses the metathesis of ethylene with unsaturated fats and oils (e.g., oleic sunflower oils, oleic rapeseed oils, and mono-alcohol esters thereof) in the presence of a ruthenium metathesis catalyst.

The α-alkene can include 1-propene, 1-butene, 1-pentene, 1-hexene, 1-heptene, 1-octene, and higher alkenes. PCT Patent Application No. WO 2008/046106, discloses the metathesis of terminal alkenes with fats and oils (e.g., soybean oil, sunflower oil, canola oil, safflower oil, cottonseed oil, castor oil, rapeseed oil, peanut oil, corn oil, olive oil, palm oil, sesame oil, grape seed oil) to form linear metathesis products using a ruthenium alkylidene catalyst. PCT Patent Application No. WO 2009/020667, discloses a method for improving catalyst efficiency by chemically treating a natural feedstock before introducing the metathesis catalyst to reduce the amount of catalyst poison. Similarly internal olefins can be used as above to generate a wider variety of even and odd chain length esters. The final unsaturated esters obtained from the above metathesis can be partially hydrogenated or fully hydrogenated to give mono-unsaturated esters or saturated esters for preparation of the sugar amides.

Furthermore, esters can also be branched as disclosed in WO 2011/088089A1 and WO 2012/009525A2.

In the above manner various mixed chain lengths, even and odd, branched or non-branched, saturated or unsaturated or mixtures of any of the above can be obtained for use in making the sugar amides. Other sources of even and odd chain length fatty acids or esters are disclosed in WO 2012/135760, US2011/0146142, EP2480680, US2010/0071259A1, and US2012/0070868. The above metathesis chemistry can also provide unique chain combinations that can be skewed, bimodal, peaked to allow the formulator to tune the type of properties they wish to obtain from use of the sugar amide compositions in various detergent applications. Examples in the literature where such combinations can potentially add additional performance vectors are disclosed in production of unique alkylbenzene sulfonate surfactants by Scheibel et al., US 2012/0214724A1. Examples of other developments in the surfactant industry are described in the Scheibel JSD Article. The potential for sugar amide combinations, however, is not limited to C₁₀-C₁₃ combinations as disclosed in the above cited reference. The sugar amide functional group allows for a much wider range of chain length options and unique chain combinations. The unique hydrophobic tail combinations described above can also allow for unique properties for non mixed sugar head groups such as Formula I or Formula II alone.

The mixture can also contain additional chemicals beyond those shown in Formulas I and II. In one embodiment, the mixture further comprises a third chemical, said third chemical having the chemical structure of Formula IV: 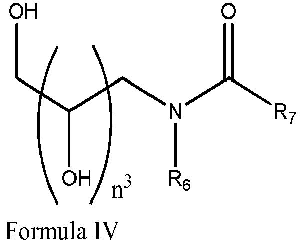 wherein R₆ is selected from hydrogen, C₁-C₁₆ alkyl, C₁-C₃ hydroxy- or methoxy-alkyl; R₇ is independently selected from C₇-C₂₃ alkyl, mono-alkenyl, di-alkenyl, or tri-alkenyl and mixtures thereof; and n³ is 2 to 4.

According to one embodiment, n³ is equal to 4. The chemical fragment in Formula IV not including the nitrogen atom, R₆ or R₇ has 6 carbon and 5 oxygen atoms. This chemical fragment can be derived from a six carbon sugar including but not limited to glucose, mannose or galactose and mixture thereof, provided it is not the same fragment derived from a sugar found in Formula I. For the avoidance of doubt, one embodiment of such a mixture comprises: a chemical of Formula I that contains a fragment derived from glucose, a chemical of Formula II that contains a fragment derived from xylose and a chemical of Formula IV that contains a fragment derived from mannose or galactose or mixtures of mannose and galactose.

According to a second embodiment, n³ is equal to 3. The chemical fragment in Formula IV not including the nitrogen atom, carbonyl, R₆ or R₇ has 5 carbon, and 4 oxygen atoms. This chemical fragment can be derived from a five carbon sugar including but not limited to xylose or arabinose and mixtures thereof provided it is not the same fragment derived from a sugar found in Formula I or Formula II. For the avoidance of doubt, one embodiment of such a mixture comprises: a chemical of Formula I that contains a fragment derived from glucose, a chemical of Formula II that contains a fragment derived from xylose and a chemical of Formula IV that contains a fragment derived from arabinose.

According to another embodiment, n¹ is equal to 4, n² is equal to 1 and n³ is 2 to 4. The chemical fragment in Formula I not including the nitrogen atom, R₁ or R₂ has 6 carbon and 5 oxygen atoms. This chemical fragment can be derived from a six carbon sugar including but not limited to glucose, mannose or galactose. The chemical fragment in Formula II not including the nitrogen atom, R₃ or R₄ has 3 carbon, and 2 oxygen atoms. This chemical fragment can be derived from a three carbon sugar or polyol including but not limited to glyceraldehyde or glycerol. The chemical fragment in Formula IV not including the nitrogen atom, carbonyl, R₆ or R₇ has 6 carbon and 5 oxygen atoms, 5 carbon and 4 oxygen atoms, or 4 carbon and 3 oxygen atoms. The chemical fragment in Formula IV can be derived from a six carbon sugar including but not limited to glucose, mannose or galactose and mixtures thereof, provided it is not the same fragment derived from a sugar found in Formula I. The chemical fragment in Formula IV can be derived from a five carbon sugar including but not limited to xylose and arabinose and mixtures thereof. The chemical fragment in Formula IV can be derived from a four carbon sugar. For the avoidance of doubt, one embodiment of such a mixture comprises: a chemical of Formula I that contains a fragment derived from glucose, a chemical of Formula II that contains a fragment derived from glycerol, and a chemical of Formula IV that contains a fragment derived from xylose, arabinose, mannose or galactose and mixtures thereof.

The processes to make sugars as well as the processes to make sugar amine and sugar amide structures have the potential to also make additional chemicals simultaneously. The additional chemicals may or may not be separated from the sugars, sugar amines, or sugar amides, and thus could be additional chemicals in the mixture. Additional chemicals that could be found in the mixture include sugar alcohols such as but not limited to glycerol, sorbitol, xylitol, dehydrated sugar alcohols such as but not limited to anhydrosorbitols, sorbitan and isosorbide; furan species, such as but not limited to furfural, furfural alcohol, hydroxymethylfurfural and dihydroxymethyl furan; tetrahydrofuran species such as but not limited to tetrahydrofurfuryl alcohol, and 2,5-bis(hydroxymethyl)tetrahydrofuran; an organic acid in either an acidic or neutralized form, such as but not limited to acetic acid, sodium acetate, ammonium acetate, or methyl ammonium acetate; monosaccharides; disaccharides; oligosaccharides; lignin residue including but not limited to phenolics; additional components derived from an enzymatic or microbial demixture; or a component with the chemical structure of either Formula I or Formula II with the proviso that an additional monosaccharide including but not limited to glucose, galactose, manose, xylose or arabinose, is attached to the primary alcohol in place of the hydrogen with a glycosidic bond.

### Consumer Products comprising Sugar Amide Surfactants

The surfactants of the present invention are useful for producing consumer products. Examples of these consumer products include cleaning and/or treatment compositions, fabric and/or hard surface cleaning and/or treatment compositions, fabric care compositions such as laundry detergents, softergents, hard surface cleaners, bar soaps, fabric softeners, special purpose cleaners, dishwashing detergents, and personal care compositions, including shampoos and conditioners. In one embodiment of the present invention, the composition has a single or multicompartment unit dose form.

Softergents include various granular or liquid softening-through-the wash types of product and can include organic (e.g., quaternary) or inorganic (e.g., clay) softeners (see, e.g., U.S. Patent Nos. 4,140,641; 4,639,321; 4,751,008; 4,844,821; 4,844,824; 4,873,001; 4,911,852; and 5,017,296; EP 753,569A; EP 315,126; and EP 422,787).

Hard surface cleaners include all-purpose cleaners, such as, for example, cream cleansers, liquid cleaners, and spray cleaners (e.g., glass cleaners, tile cleaners, bleach spray cleaners); and bathroom cleaners (e.g., mildew-removing, bleach-containing, antimicrobial, acidic type, neutral type, basic types). See, for example, EP 743,280A; EP 743,279A, and WO 96/34938 A. Bar soaps include laundry bars. The bar soaps encompass both the synthetic detergent (i.e., syndet) type, the soap-based type, and types with softener (see, e.g., WO 96/35772A; U.S. Patent No. 5,500,137; and WO 96/01889). These compositions can include those made by common soap-making techniques, such as plodding, and/or more unconventional techniques, such as casting, absorption of surfactant into a porous support, or the like. Other bar soaps, such as those described in BR 9502668; WO 96/04361A; WO 96/04360A; and U.S. Patent No. 5,540,852, as well as other hand wash detergents, such as those described in GB 2,292,155 A and WO 96/01306 A. Fabric softeners include both the conventional liquid and liquid concentrate types (see, e.g., EP 754,749A; WO 96/21715A; EP 705,900A; U.S. Patent Nos. 5,531,910 and 5,500,13 8), as well as dryer-added or substrate-supported types (see, e.g., U.S. Patent Nos. 5,562,847 and 5,559,088; and EP 704,522A). Other fabric softeners include solids, as described in, for example, U.S. Patent No. 5,505 866. Special purpose cleaners include home dry cleaning systems (see, e.g., WO 96/30583A; WO 96/30472A; WO 96/30471A; U.S. Patent No. 5,547,476; WO 96/37652 A); bleach pretreatment products for laundry (see, e.g., EP 751,210 A); fabric care pretreatment products (see, e.g., EP 752,469 A); liquid fine fabric detergent types, especially the high-foaming variety; rinse-aids for dishwashing; liquid bleaches including both chlorine type and oxygen bleach type; disinfecting agents; car or carpet cleaners or shampoos (see, e.g., EP 751,213A; WO 96/15308A); metal cleaners; cleaning auxiliaries (e.g., bleach additives, stain-sticks, pre-treatments including special foam type cleaners, as described in EP 753,560A; EP 753,559A; EP 753,558A; EP 753,557A; EP 753,556A, each incorporated herein by reference); and anti-sunfade treatments (see, e.g., WO 96/03486A; WO 96/03481A; WO 96/03369A, each incorporated herein by reference).

Consumer product cleaning compositions, can be formulated into a wide range of forms including, for example, powders, liquids, granules, gels, pastes, tablets, pouches, bars, types delivered in dual-compartment containers, spray or foam detergents and other homogeneous or multiphasic consumer cleaning product forms.

The consumer product compositions of the invention can be applied by hand in unitary or freely alterable dosage, or by automatic dispensing means. The consumer product compositions of the invention are useful in appliances, (e.g., washing machines, dishwashers), in institutional cleaning contexts (e.g., personal cleansing in public facilities), for bottle washing, for surgical instrument cleaning, and/or for cleaning electronic components. The consumer product compositions of the invention can have a wide pH range (e.g., about 2 to about 12, or higher), and a wide range of alkalinity reserve. For example, the consumer product compositions of the invention can be used in very high alkalinity reserves, such as drain unblocking, in which tens of grams of NaOH equivalent can be present per 100 grams of formulation. These mixtures can also be used in medium alkalinity reserves having 1 to 10 grams of NaOH equivalent, and mild or low-alkalinity ranges (e.g, liquid hand cleaners; acidic, hard-surface cleaners). Both high-foaming and low-foaming detergent types are encompassed.

Personal care compositions, which can be aqueous or anhydrous, are described in European Patent No. 1299080, U.S. Patent Application Publication No. 2009/0232873, and U.S. Patent No. 5,932,202. Nonlimiting examples of personal care products include those intended for use with hair or skin such as a shampoo, a hair conditioner, a hair treatment, a facial soap, a body wash, a body soap (liquid or bar), a foam bath, a make-up remover, a skin care product, an acne control product, a deodorant, an antiperspirant, a shaving aid, a cosmetic, a depilatory, a fragrance, special purpose cleaners and mixtures thereof. See, e.g., WO 96/37595A; WO 96/37592A; WO 96/37591A; WO 96/37589A; WO 96/37588A; GB 2,297,975A; GB 2,297,762A; GB 2,297,761A; WO 96/17916A; WO 96/12468A, each incorporated herein by reference. Personal care cleaning compositions can be formulated into, for example, a wipe, a cloth, a bar, a liquid, a powder, a crème, a lotion, a spray, an aerosol, a foam, a mousse, a serum, a capsule, a gel, an emulsion, a doe foot, a roll-on applicator, a stick, a sponge, an ointment, a paste, an emulsion spray, a tonic, a cosmetic, and mixtures thereof. Products, such as devices, appliances, applicators, implements, combs, brushes, and substrates are also encompassed by the invention. These products can be used alone on the skin or hair, or in combination with the personal care cleaning compositions described herein.

The personal care product of the invention can be applied by hand in unitary or freely alterable dosage, or by automatic dispensing means. The personal care composition of the invention also can be dispensed from an article, such as, for example, a bottle, a jar, a tube, a sachet, a pouch, a container, a tottle, a vial, an ampule, or a compact, or can be integrally contained within a delivery form, such as a wipe.

In some preferred embodiments, the personal care compositions of the present invention may be used in direct application to the skin or in a conventional manner for cleansing, treating or conditioning skin and hair. The compositions herein are useful for cleansing or conditioning the hair and scalp, and other areas of the body and for any other area of skin in need of treatment. The present invention may be used for treating, cleansing, or conditioning of the skin or hair of animals as well. An effective amount of the composition, typically from about 1 g to about 50 g, preferably from about 1 g to about 20 g of the composition, for cleansing and/or conditioning hair, skin or other area of the body, is topically applied to the hair, skin or other area that has preferably been wetted, generally with water, and then rinsed off. Application to the hair typically includes working the composition through the hair.

Conditioning agents, and in particular silicones, may be included in the consumer product composition. The conditioning agents useful in the compositions of the present invention typically comprise a water insoluble, water dispersible, non-volatile, liquid that forms emulsified, liquid particles. Suitable conditioning agents for use in the composition are those conditioning agents characterized generally as silicones (e.g., silicone oils, cationic silicones, silicone gums, high refractive silicones, and silicone resins), organic conditioning oils (e.g., hydrocarbon oils, polyolefins, and fatty esters) or combinations thereof, or those conditioning agents which otherwise form liquid, dispersed particles in the aqueous surfactant matrix herein. Such conditioning agents should be physically and chemically compatible with the essential components of the composition, and should not otherwise unduly impair product stability, aesthetics or performance.

The concentration of the conditioning agent in the composition should be sufficient to provide the desired conditioning benefits. Such concentration can vary with the conditioning agent, the conditioning performance desired, the average size of the conditioning agent particles, the type and concentration of other components, and other like factors.

The concentration of the silicone conditioning agent typically ranges from about 0.01% to about 10%. Non-limiting examples of suitable silicone conditioning agents, and optional suspending agents for the silicone, are described in U.S. Reissue Pat. No. 34,584, U.S. Pat. Nos. 5,104,646; 5,106,609; 4,152,416; 2,826,551; 3,964,500; 4,364,837; 6,607,717; 6,482,969; 5,807,956; 5,981,681; 6,207,782; 7,465,439; 7,041,767; 7,217,777; US Patent Application Nos. 2007/0286837A1; 2005/0048549A1; 2007/0041929A1; British Pat. No. 849,433; German Patent No. DE 10036533, which are all incorporated herein by reference; Chemistry and Technology of Silicones, New York: Academic Press (1968); General Electric Silicone Rubber Product Data Sheets SE 30, SE 33, SE 54 and SE 76; Silicon Compounds, Petrarch Systems, Inc. (1984); and in Encyclopedia of Polymer Science and Engineering, vol. 15, 2d ed., pp 204-308, John Wiley & Sons, Inc. (1989).

The compositions of the present invention may also comprise from about 0.05% to about 3% of at least one organic conditioning oil as the conditioning agent, either alone or in combination with other conditioning agents, such as the silicones (described herein). Suitable conditioning oils include hydrocarbon oils, polyolefins, and fatty esters. Also suitable for use in the compositions herein are the conditioning agents described by the Procter & Gamble Company in U.S. Pat. Nos. 5,674,478, and 5,750,122, incorporated herein by reference. Also suitable for use herein are those conditioning agents described in U.S. Pat. Nos. 4,529,586, 4,507,280, 4,663,158, 4,197,865, 4,217, 914, 4,381,919, and 4,422, 853, which are all incorporated herein by reference.

The consumer product compositions of the present invention may also contain an anti-dandruff agent. Suitable, non-limiting examples of anti-dandruff actives include: antimicrobial actives, pyridinethione salts, azoles, selenium sulfide, particulate sulfur, keratolytic acid, salicylic acid, octopirox (piroctone olamine), coal tar, and combinations thereof. Pyridinethione anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982, which are all incorporated herein by reference.

The consumer product compositions of the present invention may contain a humectant. The humectants herein are selected from the group consisting ofpolyhydric alcohols, water soluble alkoxylated nonionic polymers, and mixtures thereof. The humectants, when used herein, are preferably used at levels of from about 0.1% to about 20%, more preferably from about 0.5% to about 5%.

The consumer product compositions of the present invention may further comprise a suspending agent at concentrations effective for suspending water-insoluble material in dispersed form in the compositions or for modifying the viscosity of the composition. Such concentrations range from about 0.1% to about 10%, preferably from about 0.3% to about 5.0%.

Suspending agents useful herein include anionic polymers and nonionic polymers (e.g., vinyl polymers, acyl derivatives, long chain amine oxides, and mixtures thereof, alkanol amides of fatty acids, long chain esters of long chain alkanol amides, glyceryl esters, primary amines having a fatty alkyl moiety having at least about 16 carbon atoms, secondary amines having two fatty alkyl moieties each having at least about 12 carbon atoms). Examples of suspending agents are described in U.S. Pat. No. 4,741,855, incorporated herein by reference.

The consumer product formulations of the present invention can be in the form of pourable liquids (under ambient conditions). Such compositions will therefore typically comprise an aqueous carrier, which is present at a level of from about 20% to about 95%, more preferably from about 60% to about 85%. The aqueous carrier may comprise water, or a miscible mixture of water and organic solvent, but preferably comprises water with minimal or no significant concentrations of organic solvent, except as otherwise incidentally incorporated into the composition as minor ingredients of other essential or optional components.

The carrier useful in the present invention includes water and water solutions of lower alkyl alcohols and polyhydric alcohols. The lower alkyl alcohols useful herein are monohydric alcohols having 1 to 6 carbons, more preferably ethanol and isopropanol. The polyhydric alcohols useful herein include propylene glycol, hexylene glycol, glycerin, and propane diol.

The consumer product compositions may optionally comprise particles. The particles may be dispersed water-insoluble particles. The particles may be inorganic, synthetic, or semisynthetic. In one embodiment, the particles have an average mean particle size of less than about 300 µm.

The above cationic surfactants, together with high melting point fatty compounds and an aqueous carrier, may form a gel matrix in the composition of the present invention.

The gel matrix is suitable for providing various conditioning benefits such as slippery feel during the application to wet hair and softness and moisturized feel on dry hair. In view of providing the above gel matrix, the cationic surfactant and the high melting point fatty compound are contained at a level such that the weight ratio of the cationic surfactant to the high melting point fatty compound is in the range of, preferably from about 1:1 to about 1:10, more preferably from about 1:1 to about 1:6.

The consumer product compositions may comprise at least one skin care active, useful for regulating and/or improving the condition and/or appearance of mammalian skin. The skin care active may be soluble in oil or water, and may be present primarily in the oil phase and/or in the aqueous phase. Suitable actives include, but are not limited to, vitamins (e.g., from about 0.001% to about 10%), peptides (e.g., from about 1 x 10-7% to about 20%), sugar amines (e.g., from about 0.01% to about 15%), sunscreens (e.g., from about 1% to about 20%), oil control agents (e.g., from about 0.0001% to about 15%), tanning actives (e.g., 0.1% to about 20%), anti-acne actives (see, e.g., U. S. Patent No. 5,607,980, incorporated herein by reference; and "Antiacne Agents" in the Personal Care Product Council's International Cosmetic Ingredient Dictionary and Handbook, 13th Ed.) desquamation actives (e.g., from about 0.01% to about 10%), see, e.g., U.S. Patent Nos. 5,681,852; 5,652,228, incorporated herein by reference), anti-cellulite actives (from about 0.1% to about 10%), chelating agents (see e.g., U.S. Patent No. 5,487,884, International Publication Nos. WO91/16035 and WO91/16034, incorporated herein by reference), skin lightening agents (e.g., from about 0.1% to about 10%), flavonoids (see, e.g., U.S. Patent 6,235,773, incorporated herein by reference), protease inhibitors, non-vitamin antioxidants and radical scavengers, hair growth regulators, anti-wrinkle actives, anti-atrophy actives, minerals, phytosterols and/or plant hormones, tyrosinase inhibitors, anti-inflammatory agents, Nacyl amino acid compounds, antimicrobials, and antifungals (see e.g., U.S. application publication No. US 2006/0275237A1 and US 2004/0175347A1, incorporated herein by reference).

The surfactants of the present invention may also be used in cosmetic compositions, i.e., in products suitable for use in, on, or around the eyes, eyebrows, face, neck, chest, lips, hands, feet, or nails. Exemplary cosmetic products include eye liners, eye shadows, eyebrow pencils, mascaras, eye makeup removers, false eyelashes, under-eye concealers, eye creams, concealers, correctors, primers, blushes, bronzers, highlighters, shimmers, foundations, powders, sunscreens, brushes, face creams, lip primers, lip pencils, lipsticks, lip glosses, lip balms, lip stains, lip creams, and lotions. The compositions of the present invention may be combined with materials commonly found in these compositions, such as alkyl dimethicone copolyols, polyols, hydrophilic skin treatment agents, carriers, thickening agent (such as solid waxes, gelling agents, inorganic thickeners, oil soluble polymers, fatty compounds, and mixtures thereof), pigments, film forming agents, preservatives, vitamins, etc. Examples of cosmetic products are found in U.S. Pat. Nos. 6,325,995; 6,696,049; 6,503,495; 7,270,828, which are all incorporated herein by reference.

The consumer product compositions of the present invention may contain also vitamins and amino acids such as: water soluble vitamins and their derivatives, water soluble amino acids and their salts and/or derivatives, water insoluble amino acids viscosity modifiers, dyes, nonvolatile solvents or diluents (water soluble and insoluble), pearlescent aids, foam boosters, additional surfactants or nonionic cosurfactants, pediculocides, pH adjusting agents, perfumes, preservatives, chelants, proteins, skin active agents, sunscreens, UV absorbers, vitamins, niacinamide, caffeine and minoxidil..

The consumer product compositions of the present invention may also contain pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C.I. Names. The compositions of the present invention may also contain antimicrobial agents which are useful as cosmetic biocides.

The consumer product compositions of the present invention may also contain chelating agents.

This list of aforementioned personal care additives is not meant to be exclusive, and other components can be used.

### Packaging for the Consumer Products

Commercially marketed executions of the consumer products can be packaged in any suitable container including those constructed from paper, cardboard, plastic materials and any suitable laminates. A preferred packaging execution is described in European Application No. 94921505.7, incorporated herein by reference.

### Adjunct Materials

While not essential for the purposes of the present invention, the non-limiting list of adjuncts illustrated hereinafter are suitable for use in consumer products and may be desirably incorporated in certain embodiments of the invention, for example to assist or enhance cleaning performance, for treatment of the substrate to be cleaned, or to modify the aesthetics of the consumer product as is the case with perfumes, colorants, dyes or the like. The levels of any such adjuncts incorporated in any fabric and home care product are in addition to any materials previously recited for incorporation. The precise nature of these additional components, and levels of incorporation thereof, will depend on the physical form of the consumer product and the nature of the cleaning operation for which it is to be used. Suitable adjunct materials include, but are not limited to, surfactants, builders, chelating agents, dye transfer inhibiting agents, dispersants, enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, hueing dyes, perfumes, perfume delivery systems, structure elasticizing agents, fabric softeners, carriers, hydrotropes, processing aids, solvents and/or pigments. In addition to the disclosure below, suitable examples of such other adjuncts and levels of use are found in U.S. Patent Nos. 5,576,282,6,306,812 B1 and 6,326,348 B1 that are incorporated by reference.

As stated, the adjunct ingredients are not essential to Applicants' consumer products. Thus, certain embodiments of Applicants' consumer products do not contain one or more of the following adjuncts materials: surfactants, builders, chelating agents, dye transfer inhibiting agents, dispersants, additional enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, perfume delivery systems, structure elasticizing agents, fabric softeners, carriers, hydrotropes, processing aids, solvents and/or pigments. However, when one or more adjuncts are present, such one or more adjuncts may be present as detailed below:

### Suitable Fabric Hueing Agents

The composition may comprise a fabric hueing agent. Suitable fabric hueing agents include dyes, dye-clay conjugates, and pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof.

In another aspect, suitable small molecule dyes include small molecule dyes selected from the group consisting of Colour Index (Society of Dyers and Colourists, Bradford, UK) numbers Direct Violet 9, Direct Violet 35, Direct Violet 48, Direct Violet 51, Direct Violet 66, Direct Violet 99, Direct Blue 1, Direct Blue 71, Direct Blue 80, Direct Blue 279, Acid Red 17, Acid Red 73, Acid Red 88, Acid Red 150, Acid Violet 15, Acid Violet 17, Acid Violet 24, Acid Violet 43, Acid Red 52, Acid Violet 49, Acid Blue 15, Acid Blue 17, Acid Blue 25, Acid Blue 29, Acid Blue 40, Acid Blue 45, Acid Blue 75, Acid Blue 80, Acid Blue 83, Acid Blue 90 and Acid Blue 113, Acid Black 1, Basic Violet 1, Basic Violet 3, Basic Violet 4, Basic Violet 10, Basic Violet 35, Basic Blue 3, Basic Blue 16, Basic Blue 22, Basic Blue 47, Basic Blue 66, Basic Blue 75, Basic Blue 159 and mixtures thereof. In another aspect, suitable small molecule dyes include small molecule dyes selected from the group consisting of Colour Index (Society of Dyers and Colourists, Bradford, UK) numbers Acid Violet 17, Acid Violet 43, Acid Red 52, Acid Red 73, Acid Red 88, Acid Red 150, Acid Blue 25, Acid Blue 29, Acid Blue 45, Acid Blue 113, Acid Black 1, Direct Blue 1, Direct Blue 71, Direct Violet 51 and mixtures thereof. In another aspect, suitable small molecule dyes include small molecule dyes selected from the group consisting of Colour Index (Society of Dyers and Colourists, Bradford, UK) numbers Acid Violet 17, Direct Blue 71, Direct Violet 51, Direct Blue 1, Acid Red 88, Acid Red 150, Acid Blue 29, Acid Blue 113 or mixtures thereof.

Suitable polymeric dyes include polymeric dyes selected from the group consisting of polymers containing conjugated chromogens (dye-polymer conjugates) and polymers with chromogens co-polymerized into the backbone of the polymer and mixtures thereof.

In another aspect, suitable polymeric dyes include polymeric dyes selected from the group consisting of fabric-substantive colorants sold under the name of Liquitint® (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. In still another aspect, suitable polymeric dyes include polymeric dyes selected from the group consisting of Liquitint® (Milliken, Spartanburg, South Carolina, USA) Violet CT, carboxymethyl cellulose (CMC) conjugated with a reactive blue, reactive violet or reactive red dye such as CMC conjugated with C.I. Reactive Blue 19, sold by Megazyme, Wicklow, Ireland under the product name AZO-CM-CELLULOSE, product code S-ACMC, alkoxylated triphenyl-methane polymeric colourants, alkoxylated thiophene polymeric colourants, and mixtures thereof.

Suitable dye clay conjugates include dye clay conjugates selected from the group comprising at least one cationic/basic dye and a smectite clay, and mixtures thereof. In another aspect, suitable dye clay conjugates include dye clay conjugates selected from the group consisting of one cationic/basic dye selected from the group consisting of C.I. Basic Yellow 1 through 108, C.I. Basic Orange 1 through 69, C.I. Basic Red 1 through 118, C.I. Basic Violet 1 through 51, C.I. Basic Blue 1 through 164, C.I. Basic Green 1 through 14, C.I. Basic Brown 1 through 23, CI Basic Black 1 through 11, and a clay selected from the group consisting of Montmorillonite clay, Hectorite clay, Saponite clay and mixtures thereof. In still another aspect, suitable dye clay conjugates include dye clay conjugates selected from the group consisting of: Montmorillonite Basic Blue B7 C.I. 42595 conjugate, Montmorillonite Basic Blue B9 C.I. 52015 conjugate, Montmorillonite Basic Violet V3 C.I. 42555 conjugate, Montmorillonite Basic Green G1 C.I. 42040 conjugate, Montmorillonite Basic Red R1 C.I. 45160 conjugate, Montmorillonite C.I. Basic Black 2 conjugate, Hectorite Basic Blue B7 C.I. 42595 conjugate, Hectorite Basic Blue B9 C.I. 52015 conjugate, Hectorite Basic Violet V3 C.I. 42555 conjugate, Hectorite Basic Green G1 C.I. 42040 conjugate, Hectorite Basic Red R1 C.I. 45160 conjugate, Hectorite C.I. Basic Black 2 conjugate, Saponite Basic Blue B7 C.I. 42595 conjugate, Saponite Basic Blue B9 C.I. 52015 conjugate, Saponite Basic Violet V3 C.I. 42555 conjugate, Saponite Basic Green G1 C.I. 42040 conjugate, Saponite Basic Red R1 C.I. 45160 conjugate, Saponite C.I. Basic Black 2 conjugate and mixtures thereof.

Suitable pigments include pigments selected from the group consisting of flavanthrone, indanthrone, chlorinated indanthrone containing from 1 to 4 chlorine atoms, pyranthrone, dichloropyranthrone, monobromodichloropyranthrone, dibromodichloropyranthrone, tetrabromopyranthrone, perylene-3,4,9,10-tetracarboxylic acid diimide, wherein the imide groups may be unsubstituted or substituted by C1-C3 -alkyl or a phenyl or heterocyclic radical, and wherein the phenyl and heterocyclic radicals may additionally carry substituents which do not confer solubility in water, anthrapyrimidinecarboxylic acid amides, violanthrone, isoviolanthrone, dioxazine pigments, copper phthalocyanine which may contain up to 2 chlorine atoms per molecule, polychloro-copper phthalocyanine or polybromochloro-copper phthalocyanine containing up to 14 bromine atoms per molecule and mixtures thereof.

In another aspect, suitable pigments include pigments selected from the group consisting of Ultramarine Blue (C.I. Pigment Blue 29), Ultramarine Violet (C.I. Pigment Violet 15) and mixtures thereof.

The aforementioned fabric hueing agents can be used in combination (any mixture of fabric hueing agents can be used). Suitable fabric hueing agents can be purchased from Aldrich, Milwaukee, Wisconsin, USA; Ciba Specialty Chemicals, Basel, Switzerland; BASF, Ludwigshafen, Germany; Dayglo Color Corporation, Mumbai, India; Organic Dyestuffs Corp., East Providence, Rhode Island, USA; Dystar, Frankfurt, Germany; Lanxess, Leverkusen, Germany; Megazyme, Wicklow, Ireland; Clariant, Muttenz, Switzerland; Avecia, Manchester, UK and/or made in accordance with the examples contained herein. Suitable hueing agents are described in more detail in US 7,208,459 B2.

### Encapsulates

The composition may comprise an encapsulate. In one aspect, an encapsulate comprising a core, a shell having an inner and outer surface, said shell encapsulating said core.

In one aspect of said encapsulate, said core may comprise a material selected from the group consisting of perfumes; brighteners; dyes; insect repellants; silicones; waxes; flavors; vitamins; fabric softening agents; skin care agents in one aspect, paraffins; enzymes; anti-bacterial agents; bleaches; sensates; and mixtures thereof; and said shell may comprise a material selected from the group consisting of polyethylenes; polyamides; polystyrenes; polyisoprenes; polycarbonates; polyesters; polyacrylates; aminoplasts, in one aspect said aminoplast may comprise a polyureas, polyurethane, and/or polyureaurethane, in one aspect said polyurea may comprise polyoxymethyleneurea and/or melamine formaldehyde; polyolefins; polysaccharides, in one aspect said polysaccharide may comprise alginate and/or chitosan; gelatin; shellac; epoxy resins; vinyl polymers; water insoluble inorganics; silicone; and mixtures thereof.

In one aspect of said encapsulate, said core may comprise perfume.

In one aspect of said encapsulate, said shell may comprise melamine formaldehyde and/or cross linked melamine formaldehyde.

In a one aspect, suitable encapsulates may comprise a core material and a shell, said shell at least partially surrounding said core material, is disclosed. At least 75%, 85% or even 90% of said encapsulates may have a fracture strength of from about 0.2 MPa to about 10 MPa, from about 0.4 MPa to about 5MPa, from about 0.6 MPa to about 3.5 MPa, or even from about 0.7 MPa to about 3MPa; and a benefit agent leakage of from 0% to about 30%, from 0% to about 20%, or even from 0% to about 5%.

In one aspect, at least 75%, 85% or even 90% of said encapsulates may have a particle size of from about 1 microns to about 80 microns, about 5 microns to 60 microns, from about 10 microns to about 50 microns, or even from about 15 microns to about 40 microns.

In one aspect, at least 75%, 85% or even 90% of said encapsulates may have a particle wall thickness of from about 30 nm to about 250 nm, from about 80 nm to about 180 nm, or even from about 100 nm to about 160 nm.

In one aspect, said encapsulates' core material may comprise a material selected from the group consisting of a perfume raw material and/or optionally a material selected from the group consisting of vegetable oil, including neat and/or blended vegetable oils including caster oil, coconut oil, cottonseed oil, grape oil, rapeseed, soybean oil, corn oil, palm oil, linseed oil, safflower oil, olive oil, peanut oil, coconut oil, palm kernel oil, castor oil, lemon oil and mixtures thereof; esters of vegetable oils, esters, including dibutyl adipate, dibutyl phthalate, butyl benzyl adipate, benzyl octyl adipate, tricresyl phosphate, trioctyl phosphate and mixtures thereof; straight or branched chain hydrocarbons, including those straight or branched chain hydrocarbons having a boiling point of greater than about 80 °C; partially hydrogenated terphenyls, dialkyl phthalates, alkyl biphenyls, including monoisopropylbiphenyl, alkylated naphthalene, including dipropylnaphthalene, petroleum spirits, including kerosene, mineral oil and mixtures thereof; aromatic solvents, including benzene, toluene and mixtures thereof; silicone oils; and mixtures thereof.

In one aspect, said encapsulates' wall material may comprise a suitable resin including the reaction product of an aldehyde and an amine, suitable aldehydes include, formaldehyde. Suitable amines include melamine, urea, benzoguanamine, glycoluril, and mixtures thereof. Suitable melamines include, methylol melamine, methylated methylol melamine, imino melamine and mixtures thereof. Suitable ureas include, dimethylol urea, methylated dimethylol urea, urea-resorcinol, and mixtures thereof.

In one aspect, suitable formaldehyde scavengers may be employed with the encapsulates, for example, in a capsule slurry and/or added to a consumer product before, during or after the encapsulates are added to such consumer product.

Suitable capsules that can be made by following the teaching of USPA 2008/0305982 A1; and/or USPA 2009/0247449 A1. Alternatively, suitable capsules can be purchased from Appleton Papers Inc. of Appleton, Wisconsin USA.

In addition, the materials for making the aforementioned encapsulates can be obtained from Solutia Inc. (St Louis, Missouri U.S.A.), Cytec Industries (West Paterson, New Jersey U.S.A.), sigma-Aldrich (St. Louis, Missouri U.S.A.), CP Kelco Corp. of San Diego, California, USA; BASF AG of Ludwigshafen, Germany; Rhodia Corp. of Cranbury, New Jersey, USA; Hercules Corp. of Wilmington, Delaware, USA; Agrium Inc. of Calgary, Alberta, Canada, ISP of New Jersey U.S.A., Akzo Nobel of Chicago, IL, USA; Stroever Shellac Bremen of Bremen, Germany; Dow Chemical Company of Midland, MI, USA; Bayer AG of Leverkusen, Germany; Sigma-Aldrich Corp., St. Louis, Missouri, USA.

### Polymers

The consumer product may comprise one or more polymers. Examples are carboxymethylcellulose, poly(vinyl-pyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid co-polymers.

The consumer product may comprise one or more amphiphilic cleaning polymers such as the compound having the following general structure: bis((C₂H₅O)(C₂H₄O)n)(CH₃)-N⁺-CₓH₂ₓ-N⁺-(CH₃)-bis((C₂H₅O)(C₂H₄O)n), wherein n = from 20 to 30, and x = from 3 to 8, or sulphated or sulphonated variants thereof.

The consumer product may comprise amphiphilic alkoxylated grease cleaning polymers which have balanced hydrophilic and hydrophobic properties such that they remove grease particles from fabrics and surfaces. Specific embodiments of the amphiphilic alkoxylated grease cleaning polymers of the present invention comprise a core structure and a plurality of alkoxylate groups attached to that core structure. These may comprise alkoxylated polyalkylenimines, preferably having an inner polyethylene oxide block and an outer polypropylene oxide block.

Carboxylate polymer - The consumer products of the present invention may also include one or more carboxylate polymers such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da.

Soil release polymer - The consumer products of the present invention may also include one or more soil release polymers having a structure as defined by one of the following formulas (V), (VI) or (VII):

(V) -[(OCHR¹-CHR²)ₐ-O-OC-Ar-CO-]_{d}

(VI) -[(OCHR³-CHR⁴)_{b}-O-OC-sAr-CO-]ₑ

(VII) -[(OCHR⁵-CHR⁶)_{c}-OR⁷]_{f}

wherein:
a, b and c are from 1 to 200;
d, e and f are from 1 to 50;
Ar is a 1,4-substituted phenylene;
sAr is 1,3-substituted phenylene substituted in position 5 with SO₃Me;
Me is Li, K, Mg/2, Ca/2, Al/3, ammonium, mono-, di-, tri-, or tetraalkylammonium wherein the alkyl groups are C₁-C₁₈ alkyl or C₂-C₁₀ hydroxyalkyl, or mixtures thereof;
R¹, R², R³, R⁴, R⁵ and R⁶ are independently selected from H or C₁-C₁₈n- or iso-alkyl; and
R⁷ is a linear or branched C₁-C₁₈ alkyl, or a linear or branched C₂-C₃₀ alkenyl, or a cycloalkyl group with 5 to 9 carbon atoms, or a C₈-C₃₀ aryl group, or a C₆-C₃₀ arylalkyl group.

Suitable soil release polymers are polyester soil release polymers such as Repel-o-tex polymers, including Repel-o-tex SF, SF-2 and SRP6 supplied by Rhodia. Other suitable soil release polymers include Texcare polymers, including Texcare SRA100, SRA300, SRN100, SRN170, SRN240, SRN300 and SRN325 supplied by Clariant. Other suitable soil release polymers are Marloquest polymers, such as Marloquest SL supplied by Sasol.

Cellulosic polymer - The consumer products of the present invention may also include one or more cellulosic polymers including those selected from alkyl cellulose, alkyl alkoxyalkyl cellulose, carboxyalkyl cellulose, alkyl carboxyalkyl cellulose. In one aspect, the cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

### Enzymes

The consumer products can comprise one or more enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, mannanases, pectate lyases, keratinases, reductases, oxidases, phenoloxidases, lipoxygenases, ligninases, pullulanases, tannases, pentosanases, malanases, ß-glucanases, arabinosidases, hyaluronidase, chondroitinase, laccase, and amylases, or mixtures thereof. A typical combination is an enzyme cocktail that may comprise, for example, a protease and lipase in conjunction with amylase. When present in a consumer product, the aforementioned additional enzymes may be present at levels from about 0.00001% to about 2%, from about 0.0001% to about 1% or even from about 0.001% to about 0.5% enzyme protein by weight of the consumer product.

In one aspect preferred enzymes would include a protease. Suitable proteases include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:
(a) subtilisins (EC 3.4.21.62), including those derived from Bacillus, such as Bacillus lentus, B. alkalophilus, B. subtilis, B. amyloliquefaciens, Bacillus pumilus and Bacillus gibsonii described in US 6,312,936 B1, US 5,679,630, US 4,760,025, US7,262,042 and WO09/021867.
(b) trypsin-type or chymotrypsin-type proteases, such as trypsin *(e.g.,* of porcine or bovine origin), including the Fusarium protease described in WO 89/06270 and the chymotrypsin proteases derived from Cellumonas described in WO 05/052161 and WO 05/052146.
(c) metalloproteases, including those derived from Bacillus amyloliquefaciens described in WO 07/044993A2.

Preferred proteases include those derived from Bacillus gibsonii or Bacillus Lentus.

Suitable commercially available protease enzymes include those sold under the trade names Alcalase®, Savinase®, Primase®, Durazym®, Polarzyme®, Kannase®, Liquanase®, Liquanase Ultra®, Savinase Ultra®, Ovozyme®, Neutrase®, Everlase® and Esperase® by Novozymes A/S (Denmark), those sold under the tradename Maxatase®, Maxacal®, Maxapem®, Properase®, Purafect®, Purafect Prime®, Purafect Ox®, FN3® , FN4®, Excellase® and Purafect OXP® by Genencor International, those sold under the tradename Opticlean® and Optimase® by Solvay Enzymes, those available from Henkel/ Kemira, namely BLAP (sequence shown in Figure 29 of US 5,352,604 with the folowing mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D) - all from Henkel/Kemira; and KAP (Bacillus alkalophilus subtilisin with mutations A230V + S256G + S259N) from Kao.

Suitable alpha-amylases include those of bacterial or fungal origin. Chemically or genetically modified mutants (variants) are included. A preferred alkaline alpha-amylase is derived from a strain of Bacillus, such as Bacillus licheniformis, Bacillus amyloliquefaciens, Bacillus stearothermophilus, Bacillus subtilis, or other Bacillus sp., such as Bacillus sp. NCIB 12289, NCIB 12512, NCIB 12513, DSM 9375 (USP 7,153,818) DSM 12368, DSMZ no. 12649, KSM AP1378 (WO 97/00324), KSM K36 or KSM K38 (EP 1,022,334). Preferred amylases include:
(a) the variants described in WO 94/02597, WO 94/18314, WO96/23874 and WO 97/43424, especially the variants with substitutions in one or more of the following positions versus the enzyme listed as SEQ ID No. 2 in WO 96/23874: 15, 23, 105, 106, 124, 128, 133, 154, 156, 181, 188, 190, 197, 202, 208, 209, 243, 264, 304, 305, 391, 408, and 444.
(b) the variants described in USP 5,856,164 and WO99/23211, WO 96/23873, WO00/60060 and WO 06/002643, especially the variants with one or more substitutions in the following positions versus the AA560 enzyme listed as SEQ ID No. 12 in WO 06/002643:
   26, 30, 33, 82, 37, 106, 118, 128, 133, 149, 150, 160, 178, 182, 186, 193, 203, 214, 231, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484,(c) variants exhibiting at least 90% identity with SEQ ID No. 4 in WO06/002643, the wild-type enzyme from Bacillus SP722, especially variants with deletions in the 183 and 184 positions and variants described in WO 00/60060, which is incorporated herein by reference.
(d) variants exhibiting at least 95% identity with the wild-type enzyme from Bacillus sp.707 (SEQ ID NO:7 in US 6,093, 562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations.

Suitable commercially available alpha-amylases include DURAMYL®, LIQUEZYME®, TERMAMYL®, TERMAMYL ULTRA®, NATALASE®, SUPRAMYL®, STAINZYME®, STAINZYME PLUS®, FUNGAMYL® and BAN® (Novozymes A/S, Bagsvaerd, Denmark), KEMZYM® AT 9000 Biozym Biotech Trading GmbH Wehlistrasse 27b A-1200 Wien Austria, RAPIDASE®, PURASTAR®, ENZYSIZE®, OPTISIZE HT PLUS® and PURASTAR OXAM® (Genencor International Inc., Palo Alto, California) and KAM® (Kao, 14-10 Nihonbashi Kayabacho, 1-chome, Chuo-ku Tokyo 103-8210, Japan). In one aspect, suitable amylases include NATALASE®, STAINZYME® and STAINZYME PLUS® and mixtures thereof.

In one aspect, such enzymes may be selected from the group consisting of: lipases, including "first cycle lipases" such as those described in U.S. Patent 6,939,702 B1 and US PA 2009/0217464. In one aspect, the lipase is a first-wash lipase, preferably a variant of the wild-type lipase from Thermomyces lanuginosus comprising T231R and N233R mutations. The wild-type sequence is the 269 amino acids (amino acids 23 - 291) of the Swissprot accession number SwissProt 059952 (derived from Thermomyces lanuginosus (Humicola lanuginosa)). Preferred lipases would include those sold under the tradenames Lipex® and Lipolex®.

In one aspect, other preferred enzymes include microbial-derived endoglucanases exhibiting endo-beta-1,4-glucanase activity (E.C. 3.2.1.4), including a bacterial polypeptide endogenous to a member of the genus Bacillus which has a sequence of at least 90%, 94%, 97% and even 99% identity to the amino acid sequence SEQ ID NO:2 in 7,141,403B2) and mixtures thereof. Suitable endoglucanases are sold under the tradenames Celluclean® and Whitezyme® (Novozymes A/S, Bagsvaerd, Denmark).

Other preferred enzymes include pectate lyases sold under the tradenames Pectawash®, Pectaway®, Xpect® and mannanases sold under the tradenames Mannaway® (all from Novozymes A/S, Bagsvaerd, Denmark), and Purabrite® (Genencor International Inc., Palo Alto, California).

### Bleaching Agents

The consumer products of the present invention may comprise one or more bleaching agents. Suitable bleaching agents other than bleaching catalysts include photobleaches, bleach activators, hydrogen peroxide, sources of hydrogen peroxide, pre-formed peracids and mixtures thereof. In general, when a bleaching agent is used, the consumer products of the present invention may comprise from about 0.1% to about 50% or even from about 0.1% to about 25% bleaching agent by weight of the subject consumer product. Examples of suitable bleaching agents include:
(1) photobleaches for example sulfonated zinc phthalocyanine sulfonated aluminium phthalocyanines, xanthene dyes and mixtures thereof;
(2) preformed peracids: Suitable preformed peracids include, but are not limited to, compounds selected from the group consisting of percarboxylic acids and salts, percarbonic acids and salts, perimidic acids and salts, peroxymonosulfuric acids and salts, for example, Oxone®, and mixtures thereof. Suitable percarboxylic acids include hydrophobic and hydrophilic peracids having the formula R-(C=O)O-O-M wherein R is an alkyl group, optionally branched, having, when the peracid is hydrophobic, from 6 to 14 carbon atoms, or from 8 to 12 carbon atoms and, when the peracid is hydrophilic, less than 6 carbon atoms or even less than 4 carbon atoms; and M is a counterion, for example, sodium, potassium or hydrogen;
(3) sources of hydrogen peroxide, for example, inorganic perhydrate salts, including alkali metal salts such as sodium salts of perborate (usually mono- or tetra-hydrate), percarbonate, persulphate, perphosphate, persilicate salts and mixtures thereof. In one aspect of the invention the inorganic perhydrate salts are selected from the group consisting of sodium salts of perborate, percarbonate and mixtures thereof. When employed, inorganic perhydrate salts are typically present in amounts of from 0.05 to 40 wt%, or 1 to 30 wt% of the overall fabric and home care product and are typically incorporated into such fabric and home care products as a crystalline solid that may be coated. Suitable coatings include, inorganic salts such as alkali metal silicate, carbonate or borate salts or mixtures thereof, or organic materials such as water-soluble or dispersible polymers, waxes, oils or fatty soaps; and
(4) bleach activators having R-(C=O)-L wherein R is an alkyl group, optionally branched, having, when the bleach activator is hydrophobic, from 6 to 14 carbon atoms, or from 8 to 12 carbon atoms and, when the bleach activator is hydrophilic, less than 6 carbon atoms or even less than 4 carbon atoms; and L is leaving group. Examples of suitable leaving groups are benzoic acid and derivatives thereof - especially benzene sulphonate. Suitable bleach activators include dodecanoyl oxybenzene sulphonate, decanoyl oxybenzene sulphonate, decanoyl oxybenzoic acid or salts thereof, 3,5,5-trimethyl hexanoyloxybenzene sulphonate, tetraacetyl ethylene diamine (TAED) and nonanoyloxybenzene sulphonate (NOBS). Suitable bleach activators are also disclosed in WO 98/17767. While any suitable bleach activator may be employed, in one aspect of the invention the subject consumer product may comprise NOBS, TAED or mixtures thereof.

When present, the peracid and/or bleach activator is generally present in the consumer product in an amount of from about 0.1 to about 60 wt%, from about 0.5 to about 40 wt % or even from about 0.6 to about 10 wt% based on the fabric and home care product. One or more hydrophobic peracids or precursors thereof may be used in combination with one or more hydrophilic peracid or precursor thereof.

The amounts of hydrogen peroxide source and peracid or bleach activator may be selected such that the molar ratio of available oxygen (from the peroxide source) to peracid is from 1:1 to 35:1, or even 2:1 to 10:1.

Surfactants - The consumer products according to the present invention may comprise a surfactant or surfactant system wherein the surfactant can be selected from nonionic surfactants, anionic surfactants, cationic surfactants, ampholytic surfactants, zwitterionic surfactants, semi-polar nonionic surfactants and mixtures thereof. When present, surfactant is typically present at a level of from about 0.1% to about 60%, from about 1% to about 50% or even from about 5% to about 40% by weight of the subject consumer product.

Suitable anionic detersive surfactants include sulphate and sulphonate detersive surfactants.

Suitable sulphonate detersive surfactants include alkyl benzene sulphonate, in one aspect, C₁₀₋₁₃ alkyl benzene sulphonate. Suitable alkyl benzene sulphonate (LAS)may be obtained, by sulphonating commercially available linear alkyl benzene (LAB); suitable LAB includes low 2-phenyl LAB, such as those supplied by Sasol under the tradename SASOLAB® or those supplied by Petresa under the tradename Petrelab®, other suitable LAB include high 2-phenyl LAB, such as those supplied by Sasol under the tradename Hyblene®. A suitable anionic detersive surfactant is alkyl benzene sulphonate that is obtained by DETAL catalyzed process, although other synthesis routes, such as HF, may also be suitable.

Suitable sulphate detersive surfactants include alkyl sulphate, in one aspect, C₈₋₁₈ alkyl sulphate, or predominantly C₁₂ alkyl sulphate.

Another suitable sulphate detersive surfactant is alkyl alkoxylated sulphate, in one aspect, alkyl ethoxylated sulphate, in one aspect, a C₈₋₁₈ alkyl alkoxylated sulphate, in another aspect,a C₈₋₁₈ alkyl ethoxylated sulphate, typically the alkyl alkoxylated sulphate has an average degree of alkoxylation of from 0.5 to 20, or from 0.5 to 10, typically the alkyl alkoxylated sulphate is a C₈₋₁₈ alkyl ethoxylated sulphate having an average degree of ethoxylation of from 0.5 to 10, from 0.5 to 7, from 0.5 to 5 or even from 0.5 to 3.

The alkyl sulphate, alkyl alkoxylated sulphate and alkyl benzene sulphonates may be linear or branched, substituted or un-substituted.

The detersive surfactant may be a mid-chain branched detersive surfactant, in one aspect, a mid-chain branched anionic detersive surfactant, in one aspect, a mid-chain branched alkyl sulphate and/or a mid-chain branched alkyl benzene sulphonate, for example a mid-chain branched alkyl sulphate. In one aspect, the mid-chain branches are C₁₋₄ alkyl groups, typically methyl and/or ethyl groups.

In some cases it may be beneficial to combine the sugar amide surfactant with other non-ionic detersive surfactants. Suitable additional non-ionic detersive surfactants are selected from the group consisting of: C₈₋C₁₈ alkyl ethoxylates, such as, NEODOL® non-ionic surfactants from Shell; C₆-C₁₂ alkyl phenol alkoxylates wherein the alkoxylate units may be ethyleneoxy units, propyleneoxy units or a mixture thereof; C₁₂-C₁₈ alcohol and C₆-C₁₂ alkyl phenol condensates with ethylene oxide/propylene oxide block polymers such as Pluronic® from BASF; C₁₄-C₂₂ mid-chain branched alcohols; C₁₄-C₂₂ mid-chain branched alkyl alkoxylates, typically having an average degree of alkoxylation of from 1 to 30; alkylpolysaccharides, in one aspect, alkylpolyglycosides; polyhydroxy fatty acid amides; ether capped poly(oxyalkylated) alcohol surfactants; and mixtures thereof.

Suitable non-ionic detersive surfactants include alkyl polyglucoside and/or an alkyl alkoxylated alcohol.

In one aspect, non-ionic detersive surfactants include alkyl alkoxylated alcohols, in one aspect C₈₋₁₈ alkyl alkoxylated alcohol, for example a C₈₋₁₈ alkyl ethoxylated alcohol, the alkyl alkoxylated alcohol may have an average degree of alkoxylation of from 1 to 50, from 1 to 30, from 1 to 20, or from 1 to 10. In one aspect, the alkyl alkoxylated alcohol may be a C₈₋₁₈ alkyl ethoxylated alcohol having an average degree of ethoxylation of from 1 to 10, from 1 to 7, more from 1 to 5 or from 3 to 7. The alkyl alkoxylated alcohol can be linear or branched, and substituted or un-substituted.

Suitable cationic detersive surfactants include alkyl pyridinium compounds, alkyl quaternary ammonium compounds, alkyl quaternary phosphonium compounds, alkyl ternary sulphonium compounds, and mixtures thereof.

Suitable cationic detersive surfactants are quaternary ammonium compounds having the general formula VIII:

(VIII) (R⁸)(R⁹)(R¹⁰)(R¹¹)N⁺ X⁻

wherein, R⁸ is a linear or branched, substituted or unsubstituted C₆₋₁₈ alkyl or alkenyl moiety, R⁹ and R¹⁰ are independently selected from methyl or ethyl moieties, R¹¹ is a hydroxyl, hydroxymethyl or a hydroxyethyl moiety, X is an anion which provides charge neutrality, suitable anions include: halides, for example chloride; sulphate; and sulphonate. Suitable cationic detersive surfactants are mono-C₆₋₁₈ alkyl mono-hydroxyethyl di-methyl quaternary ammonium chlorides. Highly suitable cationic detersive surfactants are mono-C₈₋₁₀ alkyl mono-hydroxyethyl di-methyl quaternary ammonium chloride, mono-C₁₀₋₁₂ alkyl mono-hydroxyethyl di-methyl quaternary ammonium chloride and mono-C₁₀ alkyl mono-hydroxyethyl di-methyl quaternary ammonium chloride.

Builders - The consumer products of the present invention may comprise one or more detergent builders or builder systems. When a builder is used, the subject consumer product will typically comprise at least about 1%, from about 2% to about 60% or even from about 5% to about 10% builder by weight of the subject consumer product. The composition may even be substantially free of builder; substantially free means "no deliberately added" zeolite and/or phosphate. Typical zeolite builders include zeolite A, zeolite P and zeolite MAP. A typical phosphate builder is sodium tri-polyphosphate.

Chelating Agents - The consumer products herein may contain a chelating agent. Suitable chelating agents include copper, iron and/or manganese chelating agents and mixtures thereof. When a chelating agent is used, the subject consumer product may comprise from about 0.005% to about 15% or even from about 3.0% to about 10% chelating agent by weight of the subject consumer product. Suitable chelants include DTPA (Diethylene triamine pentaacetic acid), HEDP (Hydroxyethane diphosphonic acid), DTPMP (Diethylene triamine penta(methylene phosphonic acid)), 1,2-Dihydroxybenzene-3,5-disulfonic acid disodium salt hydrate, ethylenediamine, diethylene triamine, ethylenediaminedisuccinic acid (EDDS), N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP) and derivatives thereof.

Dye Transfer Inhibiting Agents - The consumer products of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. When present in a subject consumer product, the dye transfer inhibiting agents may be present at levels from about 0.0001% to about 10%, from about 0.01% to about 5% or even from about 0.1% to about 3% by weight of the consumer product.

Brighteners - The consumer products of the present invention can also contain additional components that may tint articles being cleaned, such as fluorescent brighteners.

The composition may comprise C.I. fluorescent brightener 260 in alpha-crystalline form having the following structure:

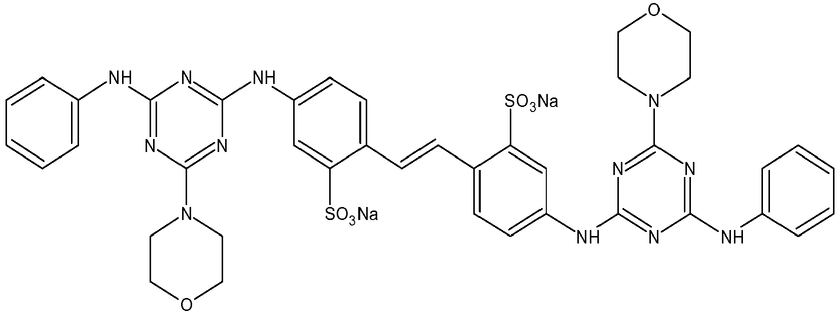

In one aspect, the brightener is a cold water soluble brightener, such as the C.I. fluorescent brightener 260 in alpha-crystalline form.

In one aspect the brightener is predominantly in alpha-crystalline form, which means that typically at least 50wt%, at least 75wt%, at least 90wt%, at least 99wt%, or even substantially all, of the C.I. fluorescent brightener 260 is in alpha-crystalline form.

The brightener is typically in micronized particulate form, having a weight average primary particle size of from 3 to 30 micrometers, from 3 micrometers to 20 micrometers, or from 3 to 10 micrometers.

The composition may comprises C.I. fluorescent brightener 260 in beta-crystalline form, and the weight ratio of: (i) C.I. fluorescent brightener 260 in alpha-crystalline form, to (ii) C.I. fluorescent brightener 260 in beta-crystalline form may be at least 0.1, or at least 0.6.

Suitable fluorescent brightener levels include lower levels of from about 0.01, from about 0.05, from about 0.1 or even from about 0.2 wt % to upper levels of 0.5 or even 0.75 wt %.

### Bleach Catalysts

The consumer products of the present invention may also include one or more bleach catalysts capable of accepting an oxygen atom from a peroxyacid and/or salt thereof, and transferring the oxygen atom to an oxidizeable substrate. Suitable bleach catalysts include, but are not limited to: iminium cations and polyions; iminium zwitterions; modified amines; modified amine oxides; N-sulphonyl imines; N-phosphonyl imines; N-acyl imines; thiadiazole dioxides; perfluoroimines; cyclic sugar ketones and mixtures thereof, as described in USPA 2007/0173430 A1.

In another aspect, the laundry detergent composition comprises a bleach ingredient, the bleach ingredient have a logP_{o/w} no greater than 0, no greater than -0.5, no greater than -1.0, no greater than -1.5, no greater than -2.0, no greater than -2.5, no greater than -3.0, or even no greater than -3.5. The method for determining logP_{o/w} is described in more detail below.

Typically, the bleach ingredient is capable of generating a bleaching species having a X_{SO} of from 0.01 to about 0.30, from 0.05 to about 0.25, or even from about 0.10 to 0.20. The method for determining X_{SO} is described in more detail below. For example, bleaching ingredients having an isoquinolinium structure are capable of generating a bleaching species that has an oxaziridinium structure. In this example, the X_{SO} is that of the oxaziridinium bleaching species.

Without wishing to be bound by theory, the inventors believe that controlling the electophilicity and hydrophobicity in this above described manner enables the bleach ingredient to be delivered substantially only to areas of the fabric that are more hydrophobic, and that contain electron rich soils, including visible chromophores, that are susceptible to bleaching by highly electrophilic oxidants.

In one aspect, the bleach catalyst has a structure corresponding to general formula IX below: 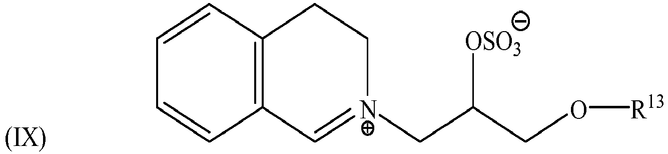 wherein R¹³ is selected from the group consisting of 2-ethylhexyl, 2-propylheptyl, 2-butyloctyl, 2-pentylnonyl, 2-hexyldecyl, n-dodecyl, n-tetradecyl, n-hexadecyl, n-octadecyl, isononyl, iso-decyl, iso-tridecyl and iso-pentadecyl;

### Method of determining logP_{o/w}

Log P_{o/w} is determined according to the method found in Brooke, D. N., Dobbs, A. J., Williams, N, Ecotoxicology and Environmental Safety (1986) 11(3): 251-260.

### Method of determining Xso

The parameter X_{SO} is determined according to the method described in Adam, W., Haas, W., Lohray, B. B. Journal of the American Chemical Society (1991) 113(16) 6202-6208.

### Silicate salts

The consumer products of the present invention can also contain silicate salts, such as sodium or potassium silicate. The composition may comprise from 0wt% to less than 10wt% silicate salt, to 9wt%, or to 8wt%, or to 7wt%, or to 6wt%, or to 5wt%, or to 4wt%, or to 3wt%, or even to 2wt%, and preferably from above 0wt%, or from 0.5wt%, or even from 1wt% silicate salt. A suitable silicate salt is sodium silicate.

### Dispersants

The consumer products of the present invention can also contain dispersants. Suitable water-soluble organic materials include the homo- or co-polymeric acids or their salts, in which the polycarboxylic acid comprises at least two carboxyl radicals separated from each other by not more than two carbon atoms.

### Enzyme Stabilizers

Enzymes for use in consumer products can be stabilized by various techniques. The enzymes employed herein can be stabilized by the presence of water-soluble sources of calcium and/or magnesium ions in the finished fabric and home care products that provide such ions to the enzymes. In case of aqueous consumer products comprising protease, a reversible protease inhibitor, such as a boron compound including borate, 4-formyl phenylboronic acid, phenylboronic acid and derivatives thereof, or compounds such as calcium formate, sodium formate and 1,2-propane diol can be added to further improve stability.

### Catalytic Metal Complexes

Applicants' compositions may include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminum cations, and a sequestrate having defined stability constants for the catalytic and auxiliary metal cations, particularly ethylenediaminetetraacetic acid, ethylenediaminetetra(methylenephosphonic acid) and water-soluble salts thereof. Such catalysts are disclosed in U.S. 4,430,243.

If desired, the compositions herein can be catalyzed by means of a manganese compound. Such compounds and levels of use are well known in the art and include, for example, the manganese-based catalysts disclosed in U.S. 5,576,282.

Cobalt bleach catalysts useful herein are known, and are described, for example, in U.S. 5,597,936; U.S. 5,595,967. Such cobalt catalysts are readily prepared by known procedures, such as taught for example in U.S. 5,597,936, and U.S. 5,595,967.

Compositions herein may also suitably include a transition metal complex of ligands such as bispidones (WO 05/042532 A1) and/or macropolycyclic rigid ligands - abbreviated as "MRLs". As a practical matter, and not by way of limitation, the compositions and processes herein can be adjusted to provide on the order of at least one part per hundred million of the active MRL species in the aqueous washing medium, and will typically provide from about 0.005 ppm to about 25 ppm, from about 0.05 ppm to about 10 ppm, or even from about 0.1 ppm to about 5 ppm, of the MRL in the wash liquor.

Suitable transition-metals in the instant transition-metal bleach catalyst include, for example, manganese, iron and chromium. Suitable MRLs include 5,12-diethyl-1,5,8,12-tetraazabicyclo[6.6.2]hexadecane.

Suitable transition metal MRLs are readily prepared by known procedures, such as taught for example in WO 00/32601, and U.S. 6,225,464.

### Solvents

Suitable solvents include water and other solvents such as lipophilic fluids. Examples of suitable lipophilic fluids include siloxanes, other silicones, hydrocarbons, glycol ethers, glycerine derivatives such as glycerine ethers, perfluorinated amines, perfluorinated and hydrofluoroether solvents, low-volatility nonfluorinated organic solvents, diol solvents, other environmentally-friendly solvents and mixtures thereof.

### Processes of Making Consumer Products

The consumer products of the present invention can be formulated into any suitable form and prepared by any process chosen by the formulator, non-limiting examples of which are described in Applicants' examples and in U.S. 4,990,280; U.S. 2003/0087791A1; U.S. 2003/0087790A1; U.S. 2005/0003983A1; U.S. 2004/0048764A1; U.S. 4,762,636; U.S. 6,291,412; U.S. 2005/0227891A1; EP 1070115A2; U.S. 5,879,584; U.S. 5,691,297; U.S. 5,574,005; U.S. 5,569,645; U.S. 5,565,422; U.S. 5,516,448; U.S. 5,489,392; U.S. 5,486,303 all of which are incorporated herein by reference.

### Methods of Use

The present invention includes methods for cleaning and/or treating a situs inter alia a surface or fabric. In one aspect, such method comprises the steps of optionally washing and/or rinsing said surface or fabric, contacting said surface or fabric with any consumer product disclosed in this specification then optionally washing and/or rinsing said surface or fabric is disclosed.

As used herein, washing includes but is not limited to, scrubbing, and mechanical agitation. Drying of such surfaces or fabrics may be accomplished by any one of the common means employed either in domestic or industrial settings. Such means include but are not limited to forced air or still air drying at ambient or elevated temperatures at pressures between 5 and 0.01 atmospheres in the presence or absence of electromagnetic radiation, including sunlight, infrared, ultraviolet and microwave irradiation. In one aspect, said drying may be accomplished at temperatures above ambient by employing an iron wherein, for example, said fabric may be in direct contact with said iron for relatively short or even extended periods of time and wherein pressure may be exerted beyond that otherwise normally present due to gravitational force. In another aspect, said drying may be accomplished at temperatures above ambient by employing a dryer. Apparatus for drying fabric is well known and it is frequently referred to as a clothes dryer. In addition to clothes such appliances are used to dry many other items including towels, sheets, pillowcases, diapers and so forth and such equipment has been accepted as a standard convenience in many nations of the world substantially replacing the use of clothes lines for drying of fabric. Most dryers in use today use heated air which is passed over and or through the fabric as it is tumbled within the dryer. The air may be heated, for example, either electronically, via gas flame, or even with microwave radiation. Such air may be heated from about 15°C to about 400°C, from about 25°C to about 200°C, from about 35°C to about 100°C, or even from about 40°C to about 85°C and used in the dryer to dry a surface and/or a fabric. As will be appreciated by one skilled in the art, the cleaning compositions of the present invention are ideally suited for use in laundry applications. Accordingly, the present invention includes a method for laundering a fabric. The method comprises the steps of contacting a fabric to be laundered with a said cleaning laundry solution comprising at least one embodiment of Applicants' cleaning composition, cleaning additive or mixture thereof. The fabric may comprise most any fabric capable of being laundered in normal consumer or institutional use conditions. The solution preferably has a pH of from about 8 to about 10.5. The compositions may be employed at concentrations of from about 500 ppm to about 15,000 ppm in solution. The water temperatures typically range from about 5 °C to about 90 °C. The water to fabric ratio is typically from about 1:1 to about 30:1.

### Hand Machine Dishwashing Methods

Any suitable methods for machine washing or cleaning soiled tableware, particularly soiled silverware are envisaged.

A preferred liquid hand dishwashing method involves either the dissolution of the detergent composition into a receptacle containing water, or by the direct application of the liquid hand dishwashing detergent composition onto soiled dishware.

A preferred machine dishwashing method comprises treating soiled articles selected from crockery, glassware, hollowware, silverware and cutlery and mixtures thereof, with an aqueous liquid having dissolved or dispensed therein an effective amount of a machine dishwashing composition in accord with the invention. By an effective amount of the machine dishwashing composition it is meant from 8 g to 60 g of product dissolved or dispersed in a wash solution of volume from 3 to 10 liters, as are typical product dosages and wash solution volumes commonly employed in conventional machine dishwashing methods.

### Cleansing Hard Surfaces

Any suitable methods for cleaning hard surfaces, such as wood, ceramic, glass, marble, porcelain, grout or concrete using the compositions described herein are envisaged. In some embodiments, an effective amount of a detergent composition of the invention is directly applied to the hard surface.

### EXAMPLES

### Example 1 - Synthesis of N-Methylglucamine

A 160ml Parr reactor was charged with Raney nickel (2.7 g, 15 wt% based on D-glucose, Grace 4200) and water (20 g). The reactor was sealed, purged three times with 300 PSI N₂ followed by three times with 300 PSI H₂. The reactor was then charged with 300 PSI H₂, at which point stirring was begun at 400 RPM, and heated to 100-110°C for 1hr. The reactor and contents were cooled to ∼10°C with external cooling; stir rate was slowed to 100 RPM and vented to ∼100 PSI. Next D-glucose was added (45 g 40% aqueous solution, 100 mmoles, Amresco) followed by methyl amine (15.37 g 40% aqueous solution, 150 mmoles, Aldrich) via an HPLC pump at 5ml/min while maintaining a temperature of around 10°C. Reactor was charged to 450 PSI H₂, stir rate was increased to 400 RPM and allowed to warm to ambient temperature over 30 min. The reactor was then externally heated to 35°C for 18 hrs, 50°C for 1 hr, 75°C for 1 hr and finally 100°C for 1 hr during which time pressure was maintained at 300-500 PSI H₂. The reactor was cooled to ambient temperature, vented and purged three times with 300 PSI N₂. The contents were filtered and stripped of water under reduced pressure on a rotary-evaporator at 70°C. The resultant solid was dissolved in refluxing methanol (35 ml) and allowed to stand at ambient temperature 18 hrs to yield a white solid, which was filtered and dried to yield 12.2 g (62.5% yield); GC Analysis was conducted by derivatizing 2 mg analyte in pyridine (1.5 ml, Aldrich BioTech Grade) with 99:1 BSTFA+TMCS (0.5 ml, Supelco, Sylon BFT) at 70-80°C for 30 min. The retention time of material matched standard from Aldrich, and showed 99% pure product by area percent.

### Example 2 - Synthesis of N-Methylxylamine

The procedure of Example 1 was followed using D-xylose (22.5 g, 150 mmoles, Spectrum) 50% water in place of D-glucose. After stripping water from crude product, the resulting mixture was diluted with ethanol (100 ml) and stripped on a rotary-evaporator at 40°C. A viscous yellow syrup which did not solidify at ambient temperature resulted (24.5 g, 99% yield, 96.4% product by GC using derivatization described in Example 1). This yellow syrup was diluted with methanol (1 weight equivalent) and stored over Type 4A molecular sieves.

### Example 3 - Synthesis of N-Dodecanoyl-N-Methylglucamine (C12-NMG)

A slurry of N-Methylglucamine (40 g, 205 mmoles, Aldrich), Methyl-Laurate (44.7 g, 208 mmoles, Aldrich) and methanol (17 g, 20 wt% based on reactants) was heated to 80°C under N₂ blanket. Once attemperature, sodium methoxide (4.4 g of 25% in methanol, 20.5 mmoles, Aldrich) was added to reaction. Stirring for ∼30min at 80°C yielded a homogenous solution from which the excess methanol was removed by short-path distillation. Heating was continued for an additional 30 min at 80°C followed by 2 hrs at 90°C. The reaction mixture was then cooled to 50°C, and 200 ml methanol was added over 10 min yielding a homogenous solution. This solution was cooled and allowed to stand at ambient temperature for 18 hrs yielding a white precipitant which was collected by filtration and dried (62.4 g, 80.7% yield, 98.9% product by GC, using derivatization described in Example 1).

### Example 4 - Synthesis of N-Dodecanoyl-N-Methylxylamine (C12-NMX)

The procedure of Example 3 was used substituting N-Methylxylamine (14.9 g of 56.3 wt% solution in methanol, 50.8 mmoles) for N-Methylglucamine and using Methyl-Laurate (12 g, 95%, 52.3 mmoles). After stirring at 90°C 2 hrs, the crude product was stirred an additional 1 hr under vacuum to a final pressure of 20 mm Hg yielding a tan paste (17.3 g, 99% yield, 97.7% products by GC, using derivatization described in Example 1).

### Example 5 - Synthesis of N-Dodecanoyl-(3-methylamino-1,2-propanediol) (C12-NMGly)

The procedure of Example 3 was followed using 3-Methylamino-1,2-propanediol (16.3 g. 76.2 mmole, Aldrich) in place of N-Methylglucamine. After stirring at 90°C for 2 hrs, the crude product was poured into crystallizing dish and allowed to solidify overnight. The resulting solid was dried at room temperature (RT) under vacuum for 24 hours yielding a white solid which was used without further purification (21.7 g, 99% yield, 99% products by GC using derivatization described in Example 1).

### Example 6 - Preparation of Modified Soybean Oil via Metathesis of HOSBO w/ 1-Butene

Reaction was performed under argon in oven dried glassware was oven dried. 1,2-dichloroethane and high oleic soybean oil were stored over 4A molecular sieves and degassed with argon before use. High oleic soybean oil, HOSBO, (504 g) was added to a 1000 ml round bottom three neck flask which was fitted with a stir bar, thermocouple, gas dispersion tube, and rubber stopper. 1-butene was bubbled through the oil at a rate of 55 g/hr and after 10 mins Hoveyda-Grubbs 2^{nd} Gen catalyst (491 mg) dissolved in 1,2-dichloroethane (2.0 ml) was added via syringe. The reaction mixture was stirred for 4 hours. Catalyst was removed from the reaction mixture by three successive treatments with bleaching clay (BASF F-160, 50 g, 25 g, and 12 g) with filtering of the bleaching clay between each treatment. Olefins were removed from the modified oil by vacuum distillation to yield 331g of modified soybean oil.

### Example 7 - Preparation of Modified Soybean Oil via Metathesis of HOSBO w/ 1-Pentene

Reaction was performed under argon in oven dried glassware was oven dried. 1,2-dichloroethane and high oleic soybean oil were stored over 4A molecular sieves and degassed with argon before use. High oleic soybean oil (606 g) was added to a 2000 ml round bottom three neck flask which was fitted with a stir bar, thermocouple, and Dewar condenser. The Dewar condenser was kept cool by filling with ethylene glycol and adding pieces of dry ice to keep the temp around -5°C. 1-pentene (205 g) was added and the reaction mixture was heated to 35°C. Once heated, Hoveyda-Grubbs 2^{nd} Gen catalyst (592 mg) dissolved in 1,2-dichloroethane (2.0 ml) was slowly added via syringe. Upon addition of the catalyst the reaction mixture demonstrated an exotherm to ∼42°C and evolved gas. The reaction mixture was stirred for 2.5 hr and cooled to room temp. Catalyst was removed from the reaction mixture by three successive treatments with bleaching clay (BASF F-160, 50 g, 25 g, and 12 g) with filtering of the bleaching clay between each treatment. Olefins were removed from the modified oil by vacuum distillation to yield 449g of modified soybean oil.

### Example 8 - Preparation of Modified Soybean Oil via Metathesis of HOSBO w/ 1-Hexene

Reaction was performed under argon in oven dried glassware was oven dried. 1,2-dichloro ethane and high oleic soybean oil were stored over 4A molecular sieves and degassed with argon before use. High oleic soybean oil (612 g) was added to a 2000 ml round bottom three neck flask which was then fitted with a stir bar, thermocouple, and Dewar condenser. The Dewar condenser was kept cool by filling with ethylene glycol and adding pieces of dry ice to keep the temp around - 5°C. 1-hexene (260 g) was added and the reaction mixture was heated to 40°C. Once heated, Hoveyda-Grubbs 2^{nd} Gen catalyst (609 mg) dissolved in 1,2-dichloroethane (2.0 ml) was slowly added via syringe. Upon addition of the catalyst the reaction mixture demonstrated an exotherm to ∼45°C and evolved gas. The reaction mixture was stirred for 2.5 hr and cooled to room temp. Catalyst was removed from the reaction mixture by three successive treatments with bleaching clay (BASF F-160, 50 g, 25 g, and 12 g) with filtering of the bleaching clay between each treatment. Olefins were removed from the modified oil by vacuum distillation to yield 447g of modified soybean oil.

### Example 9 - Preparation of Modified Soybean Oil via Metathesis of HOSBO w/ 1-Heptene

Reaction was performed under argon in oven dried glassware was oven dried. 1,2-dichloroethane and high oleic soybean oil were stored over 4A molecular sieves and degassed with argon before use. High oleic soybean oil (604 g) was added to a 2000 ml round bottom three neck flask which was fitted with a stir bar, thermocouple, and Dewar condenser. The Dewar condenser was kept cool by filling with ethylene glycol and adding pieces of dry ice to keep the temp around -5°C. 1-heptene (294 g) was added and the reaction mixture was heated to 45°C. Once heated, Hoveyda-Grubbs 2^{nd} Gen catalyst (604 mg) dissolved in 1,2-dichloroethane (2.0 ml) was slowly added via syringe. Upon addition of the catalyst, the reaction mixture demonstrated an exotherm to ∼50°C and evolved gas. The reaction mixture was stirred for 2.5 hr and cooled to room temp. Catalyst was removed from the reaction mixture by three successive treatments with bleaching clay (BASF F-160, 50 g, 25 g, and 12 g) with filtering of the bleaching clay between each treatment. Olefins were removed from the modified oil by vacuum distillation to yield 473g of modified soybean oil.

### Example 10 - Preparation of Methyl Dodec-9-enoate

Under Argon, modified soybean oil (332 g) from Example 6 was added to a 1000 ml 3 neck flask and was fitted with a stir bar, condenser, and thermocouple. MeOH (135 g) was added and the reaction mixture was heated to 65°C. Once heated, NaOMe (8.9 grams of 25 wt% solution) was slowly added and the reaction mixture was stirred for 16 hr. The reaction mixture was cooled to room temperature and transferred to a separatory funnel and allowed to stand for 2 hr. The bottom layer was removed and hexane (500 ml) was added to the top ester layer. This was then washed with water (2 x 225 ml) and brine (225 ml). The organic layer was dried over MgSO₄ and the solvent removed with a rotary evaporator to yield a crude product which was purified by vacuum distillation. 23.7 g of a clear oil resulted with a purity of 91.7% mixture of trans and cis isomers as measured by GC area percent.

### Example 11 - Preparation of Methyl-Tridec-9-enoate

The procedure of example 10 was followed with the following modifications; modified soybean oil (449 g) from Example 7 was used, a 2000 ml 3 neck flask was used and NaOMe (14.6 grams of 25 wt% solution) was used. 47.9 g of a clear oil resulted with a purity of 92.4% mixture of trans and cis isomers as measured by GC area percent.

### Example 12 - Preparation of Methyl Tetradec-9-enoate:

The procedure of example 10 was followed with the following modifications; modified soybean oil (447 g) from Example 8 was used, a 2000 ml 3 neck flask was used and NaOMe (14.6 grams of 25 wt% solution) was used. 35.0 g of a clear oil resulted with a purity of 92.5% mixture of trans and cis isomers as measured by GC area percent.

### Example 13 - Preparation of Methyl Pentadec-9-enoate:

The procedure of example 10 was followed with the following modifications; modified soybean oil (473 g) from Example 9 was used, a 2000 ml 3 neck flask was used and NaOMe (14.6 grams of 25 wt% solution) was used. After distillation, the resulting material was passed through a silica plug using first hexane then 20% EtOAc in Hxn to elute. 30.5 g of a clear oil resulted with a purity of 87.2% mixture of trans and cis isomers as measured by GC area percent.

### Example 14 - Synthesis of N-(9-Dodecenoyl)-N-Methylglucamine (C12-ene-NMG)

The procedure of Example 3 was followed using Methyl Dodec-9-eneoate from Example 10 (11.5g, 50.5 mmole). Recystallization from 40ml ethanol at ambient temperature yielded a white precipitant which was collected by filtration and dried (9.9g, 52.6% yield, 97% products by GC using derivatization described in Example 1).

### Example 15 - Synthesis of N-(9-tridecenoyl)-N-Methylglucamine (C13-ene-NMG)

The procedure of Example 3 was followed using Methyl Tridec-9-eneoate from Example 11 (17.5g, 90.3 mmole). Recystallization from 50ml ethanol at -10°C yielded a white precipitant which was collected by filtration and dried (14.7g, 42.7% yield, 94.7% products by GC using derivatization described in Example 1).

### Example 16 - Synthesis of N-(9-Tetradecenoyl)-N-Methylglucamine (C14-ene-NMG)

The procedure of Example 3 was followed using Methyl Tetradec-9-eneoate from Example 12 (15.0g, 62.5 mmole). Recystallization from 50ml ethanol at -10°C yielded a white precipitant which was collected by filtration and dried (14.1g, 55.9% yield, 94.4% products by GC using derivatization described in Example 1).

### Example 17 - Synthesis of N-(9-Pentadecenoyl)-N-Methylglucamine (C15-ene-NMG)

The procedure of Example 3 was followed using Methyl Pentadec-9-eneoate from Example 13 (12.0g, 47.2 mmole). Recystallization from 25ml ethanol at -10°C yielded a white precipitant which was collected by filtration and dried (16.9g, 84.8% yield, 95.3% products by GC using derivatization described in Example 1).

### Example 18 - Synthesis of N-(9-Dodecenoyl)-N-Methylxylamine (C12-ene-NMX)

The procedure of Example 4 was followed using Methyl Dodec-9-eneoate from Example 10 (11.5g, 50.5 mmole) yielded a yellow paste (19.0g, 97% yield, 95.2% products by GC using derivatization described in Example 1).

### Example 19 - Synthesis of N-(9-tridecenoyl)-N-Methylxylamine (C13-ene-NMX)

The procedure of Example 4 was followed using Methyl Tridec-9-eneoate from Example 11 (20g, 88.5 mmole) yielded a yellow paste (31.6g, 99% yield, 97.7% products by GC using derivatization described in Example 1).

### Example 20 - Synthesis of N-(9-Tetradecenoyl)-N-Methyl-Xylamine (C14-ene-NMX)

The procedure of Example 4 was followed using Methyl Tetradec-9-eneoate from Example 12 (15.0g, 62.5 mmole) yielded a tan paste (23.5g, 100% yield, 96.5% products by GC using derivatization described in Example 1).

### Example 21 - Synthesis of N-(9-Pentadecenoyl)-N-Methyl-Xylamine (C15-ene-NMX)

The procedure of Example 4 was followed using Methyl Pentadec-9-eneoate from Example 13 (12.0g, 47.2 mmole) yielded a tan paste (18.4g, 100% yield, 96.3% products by GC using derivatization described in Example 1).

### Example 22 - C12-NMG:C12-NMX Mixed Sample Preparations

1 g samples of title material blends (ratio given by weight) were prepared by weighing appropriate amount of individual materials and combining in glass vial with methanol (15ml). The glass vials were sonicated at ambient temperature for 20 minutes to yield a homogenous solution. The solution was poured into a 65 mm X 125 mm crystallizing dish and solvent was allowed to evaporate over 2 hours at ambient temperature under hood air flow. The resultant solid was allowed to dry an additional 48 hours at ambient temperature in hood. Resultant solid was dried 6 hours at 50°C for 18 hours at ambient temperature in a vacuum oven, ground and vacuum oven dried a second time at the same conditions. The samples were then stored in glass vials under nitrogen and aged 10 days at ambient temperature prior to thermal property analysis.

### Example 23 - C12-NMG:C12-NMGly Mixed Sample Preparations

The procedure for Example 22 was followed using the title materials with vacuum drying run at ambient temperature instead of 50°C. Samples were then aged 10 days at 10°C

### Example 24 - C12-ene-NMG:C12-ene-NMX Mixed Sample Preparations

The procedure for Example 22 was followed using the title materials with vacuum drying run at ambient temperature instead of 50°C. Samples were then aged 10 days at 10°C

### Example 25 C15-ene-NMG:C15-ene-NMX Mixed Sample Preparations

The procedure for Example 22 was followed using the title materials with vacuum drying run at ambient temperature instead of 50°C. Samples were then aged 10 days at 10°C

### Example 26 - C12/14-NMG:NMX: Cellulosic Impurities Mixed Sample Preparation

The procedure of Example 1 is followed using a mixture of C5/C6 sugars derived from cellulosic material (22.5 g, 150 mmoles) 50% water in place of D-glucose. After stripping water from crude product, the resulting mixture is diluted with ethanol (100 ml) and stripped on a rotary-evaporator at 40°C. A viscous yellow syrup which does not solidify at ambient temperature is the result. This yellow syrup is diluted with methanol (1 weight equivalent) and stored over Type 4A molecular sieves. The procedure of Example 4 is followed using Commercial C12/C14 fatty methyl ester (CE1270®) from Procter & Gamble Chemicals to give the surfactant mixture.

### Example 27 - Visual Melting Point Determination of C12-NMG:C12-NMX Mixed Samples

A Kimax -51 capillary tube (size 1.5-1.8 X 90 mm) was charged with the mixed C12-NMG:C12-NMX samples described in Example 22 to afford ∼1/2 inch sample bed. A Tomas Hoover Capillary Melting Point Apparatus was used to determine melting points visually. The Starting Melting Point (MP) is defined as the temperature (°C) when the initial white opaque solid has completed the transition to a transparent gel. The Ending Melting Point (MP) is defined as the temperature (°C) when the transparent gel transitions to liquid. The following melt points were observed for specified blended sample:

### C12-NMG:C12-NMX Mixed Samples:

**Table 1. Visual Melting of C12-NMG:C12-NMX Mixtures**

| % C12-NMG | %C12-NMX | Starting MP (°C) | Ending MP (°C) |
|---|---|---|---|
| 100 | 0 | 96 | 129.8 |
| 99 | 1 | 96 | 130 |
| 95 | 5 | 95 | 128 |
| 90 | 10 | 93 | 127.5 |
| 80 | 20 | 90 | 123 |
| 70 | 30 | 87 | 119.8 |
| 60 | 40 | 83 | 116.7 |
| 50 | 50 | 69 | 112.3 |
| 40 | 60 | 68 | 110.4 |
| 30 | 70 | 67 | 106.8 |
| 20 | 80 | 76.5 | 95.6 |
| 10 | 90 | 72.1 | 99.3 |
| 0 | 100 | 76 | 96.6 |

The physical thermal properties as measured by visual melting point of 100% C12-NMG and 100% C12-NMX can be found in the data presented above. The initial melting points observed here correspond very closely to those referenced by Zhu et al. They observed values of 90.5-91.5°C for C12-NMG and 74-75°C for C12-NMX. This initial melting point according to Zhu et al and Laughlin R.G., The Aqueous Phase Behavior of Surfactants, Academic Press, Inc. Sand Diego, CA 1994, p. 303 is "the temperature at which the crystalline state disappears. The resulting liquid state may be either isotropic or a thermatropic liquid crystal." When C12-NMG is mixed with materials based on a shorter starting sugar or polyol group such as C12-NMX, the physical thermal properties are improved beyond either C12-NMG or the C12-NMX. This improvement is shown with a drop in the starting melting point to a value below either C12-NMG or C12-NMX for the C12-NMG:C12-NMX system over a range of around 40 - 90% C12-NMX.

### Example 28 - Visual Melting Point Determination of C12-NMG:C12-NMGly Mixed Samples

The procedure of Example 27 was followed using the mixed C12-NMG:C12-NMGly samples described in Example 23.

### C12-NMG:C12-NMGly Mixed Samples:

**Table 2. Visual Melting of C12-NMG:C12-NMGly Mixtures**

| % C12-NMG | % C12-NMGly | Starting MP (°C) | Ending PM (°C) |
|---|---|---|---|
| 100 | 0 | 96.8 | 127.2 |
| 75 | 25 | 87.2 | 103.9 |
| 50 | 50 | 81.6 | 85.0 |
| 25 | 75 | 40.4 | 47.0 |
| 0 | 100 | 46.3 | 52.8 |

The physical thermal properties as measured by visual melting point of 100% C12-NMG and 100% C12 NMGly can be found in the data presented above. The initial melting points observed here correspond very closely to those referenced by Zhu et al. They observed values of 90.5-91.5°C for C12-NMG and 40-40.5°C for C12-NMGly. When C12-NMG is mixed with materials based on a shorter starting sugar or polyol group such as C12-NMGly, the physical thermal properties are improved beyond either C12-NMG or the C12-NMGly. This improvement is shown with a drop in both the starting and ending melting point to a value below either C12-NMG or C12-NMGly for the C12-NMG:C12-NMGly system at around 75% C12-NMGly.

### Example 29 - Thermal Transition Determination via DSC of C12-NMG:C12-NMX Mixed Samples

A Tzero Hermetic pan & lid was charged with ∼10-15 mg mixed material sample described in Examples 22. A Direct Scanning Coulometer, DSC Q2000 V24.9 was used to determine thermal transitions. A DSC is able to detect phase transitions within a material that might not be visible to the naked eye. The following temperature profile was used to obtain the data: ambient temperature cooled to -20°C at 20°C/min, -20°C to 160°C at 20°C/min. The T intercept is defined as the temperature where the extrapolated slope of the initial curve of a peak intercepts the interpolated baseline of that peak. T Peak Max is defined as the temperature at the peak (inflection point) of a given curve. Some samples displayed multiple peaks as shown below.

### C12-NMG:C12-NMX Mixed Samples:

Temperature grogram: Ambient temperature to -20°C at 20°C/min, -20°C to 160°C at 20°C/min.

**Table 3. Thermal Transition Determination of C12-NMG:C12-NMX by DSC**

| %C12-NMG | %C12-NMX | T Intercept (°C) | T Peak Max (°C) | T Peak Max-2 (°C) | T Intercept-3 (°C) | T Peak Max-3 (°C) |
|---|---|---|---|---|---|---|
| 100 | 0 | 84.1 | 94.7 | | 136.7 | 137.9 |
| 99 | 1 | 85.5 | 95.4 | | 136.2 | 137.3 |
| 95 | 5 | 84.1 | 98.6 | | 132.7 | 134.1 |
| 90 | 10 | 83.0 | 94.0 | | 129.9 | 131.9 |
| 80 | 20 | 76.7 | 55.7 | 91.8 | 126.5 | 128.9 |
| 70 | 30 | 43.6 | 59.6 | 86.5 | 121.2 | 122.8 |
| 60 | 40 | 49.4 | 60.2 | 84.5 | 116.4 | 118.8 |
| 50 | 50 | 51.0 | 62.5 | 83.2 | 114.0 | 115.6 |
| 40 | 60 | 51.5 | 69.2 | | 110.0 | 113.0 |
| 30 | 70 | 51.5 | 68.9 | | 107.0 | 108.6 |
| 20 | 80 | 70.9 | 77.8 | | 95.2 | 96.4 |
| 10 | 90 | 64.9 | 73.8 | | 99.3 | 101.0 |
| 0 | 100 | 69.7 | 77.0 | | 95.6 | 97.2 |

Multiple phase transitions were noted for the samples shown above. The physical thermal properties of 100% C12-NMG and 100% C12 NMX as measured by DSC can be found in the data presented above. When C12-NMG is mixed with materials based on a shorter starting sugar or polyol group such as C12-NMX, the physical thermal properties are improved beyond either C12-NMG or the C12-NMX. This improvement is shown with a drop in temperature of the initial phase transition (peak 1) to a value below either C12-NMG or C12-NMX for the C12-NMG:C12-NMX system over a range of around 20 - 90% C12-NMX.

### Example 30 - Thermal Transition Determination via DSC of C12-NMG:C12-NMGly Mixed Samples

A Tzero Hermetic pan & lid was charged with ∼10-15 mg mixed material sample described in Examples 22. A Direct Scanning Coulometer, DSC Q2000 V24.9 was used to determine thermal transitions. The following temperature profile was used to obtain the data: ambient temperature cooled to -40°C at 20°C/min, -40°C to 160°C at 20°C/min. The T intercept is defined as the temperature where the extrapolated slope of the initial curve of a peak intercepts the interpolated baseline of that peak. T Peak Max is defined as the temperature at the peak (inflection point) of a given curve. Some samples displayed multiple peaks as shown below.

### C12-NMG:C12-NGly Mixed Samples:

Temperature program: Ambient temperature to -40°C at 20°C/min, -40°C to 140°C at 20°C/min.

**Table 4. Thermal Transition Determination of C12-NMG:C12-NMGly by DSC**

| %C12-NMGly | %C12-NMG | T Intercept (°C) | T Peak Max (°C) | T Intercept-2 (°C) | T Peak Max-2 (°C) |
|---|---|---|---|---|---|
| 0 | 100 | 82.0 | 94.7 | 137.0 | 139.7 |
| 25 | 75 | 67.5 | 85.7 | 107.8 | 108.5 |
| 50 | 50 | 35.4 | 47.3 | | 80.0 |
| 75 | 25 | 37.9 | 43.6 | | |
| 100 | 0 | 44.5 | 49.6 | 117.2 | 122.4 |

Multiple phase transitions were noted for the samples shown above. The physical thermal properties of 100% C12-NMG and 100% C12 NMGly as measured by DSC can be found in the data presented above. When C12-NMG is mixed with materials based on a shorter starting sugar or polyol group such as C12-NMGly, the physical thermal properties are improved beyond either C12-NMG or C12-NMGly. This improvement is shown with a drop in temperature of the initial phase transition (peak 1) to a value below either C12-NMG or C12-NMGly for the C12-NMG:C12-NMGly at 50 and 75% C12-NMGly.

### Example 31 - Thermal Transition Determination via DSC of C12-ene-NMG:C12-ene-NMX Mixed Samples

The procedure of Example 30 was followed using the mixed C12-ene-NMG:C12-ene-NMX samples described in Example 24. When placing the samples into the Tzero Hermetic pan & lid, it was noted that at ambient temperature, the samples containing 100% C12-ene-NMG and 75% C12-ene-NMG, 25% C12-ene-NMX were powders; 50% C12-ene-NMG, 50% C12-ene-NMX, and 100% C12-ene-NMX were waxy in nature; and the sample containing 25% C12-ene-NMG, 75% C12-ene-NMX was a waxy paste.

**Table 5. Thermal Transition Determination of C12-ene-NMG:C12-ene-NMX by DSC**

| %C12-ene-NMG | %C12-ene-NMX | T Intercept (°C) | T Peak Max (°C) | T Intercept-2 (°C) | T Peak Max-2 (°C) |
|---|---|---|---|---|---|
| 100 | 0 | 67.0 | 78.7 | 106.1 | 108.5 |
| 75 | 25 | 46.3 | 64.0 | 103.0 | 105.9 |
| 50 | 50 | 38.0 | 52.6 | 98.1 | 102.4 |
| 25 | 75 | ** | ** | 94.7 | 97.6 |
| 0 | 100 | 26.0 | 28.6 | 86.2 | 89.5 |

| | | | | | |
|---|---|---|---|---|---|
| ** No peak was observed for this sample | | | | | |

Multiple phase transitions were noted for the samples shown above. The physical thermal properties of C12-ene-NMG and C12-ene-NMX as measured by DSC can be found in the data presented above. Interestingly enough, unlike previous samples, the initial low temperature transition was not observed for the mixture of 25% C12-ene-NMG and 75% C12-ene-NMX. The physical sample at this composition, however, was a noticeable paste, as opposed to either a powder or hard wax as the other samples. The presence of a paste like consistency at 75% C12-ene-NMX, demonstrates improved thermal properties over the powdered solids or waxy solids of the other samples.

### Example 32 - Thermal Transition Determination via DSC of C15-ene-NMG:C15-ene-NMX Mixed Samples

The procedure of Example 30 was followed using the mixed C15-ene-NMG:C15-ene-NMX samples described in Example 25. When placing the samples into the Tzero Hermetic pan & lid, it was noted that at ambient temperature, the samples containing 50% C15-ene-NMG, 50% C15-ene-NMX, and 100% C15-ene-NMX were waxy in nature, while the sample containing 25% C15-ene-NMG, 75% C15-ene-NMX was a waxy paste.

**Table 6. Thermal Transition Determination of C15-ene-NMG:C15-ene-NMX by DSC**

| %C15-ene-NMG | %C15-ene-NMX | T Intercept (°C) | T Peak Max (°C) | T Intercept-2 (°C) | T Peak Max-2 (°C) |
|---|---|---|---|---|---|
| 100 | 0 | 64.1 | 77.9 | 144.6 | 146.2 |
| 75 | 25 | 36.9 | 65.5 | 134.3 | 135.8 |
| 50 | 50 | 35.8 | 57.5 | 131.1 | 132.2 |
| 25 | 75 | ** | ** | 122.2 | 124.5 |
| 0 | 100 | 27.5 | 38.8 | 113.0 | 115.5 |

| | | | | | |
|---|---|---|---|---|---|
| ** No peak was observed for this sample | | | | | |

Multiple phase transitions were noted for the samples shown above. The physical thermal properties of C15-ene-NMG and C15-ene-NMX as measured by DSC can be found in the data presented above. Interestingly enough, like Example 31 and unlike other examples, the initial low temperature transition was not observed for the mixture of 25% C15-ene-NMG and 75% C15-ene-NMX. The physical sample at this composition, however, was again a noticeable paste, as opposed to either a powder or hard wax as the other samples. The presence of a paste like consistency at 75% C15-ene-NMX, demonstrates improved thermal properties over the powdered solids or waxy solids of the other samples.

### Example 33 - Krafft Point Analysis of C12-NMG:C12-NMX Mixed Samples

Krafft Point analysis was performed on a Phase Technology® NK60-KPA Analyzer that used a Diffusive Light Scattering (DLS) detection method. A 1 wt% solution was prepared in deionized water and 0.150 ml of this solution was added to the sample cell of the instrument at room temperature (20-25°C). The following heating and cooling cycle was executed on the sample. Samples were warmed to 40°C at a ramp rate of 5°C/min and held for 30 seconds. Next, Samples were cooled to -20°C at a rate of -10°C/min and held for 60 seconds. Samples were then warmed to 0°C at a rate of 5°C/min, held for 0 seconds, warmed to 30°C at a rate of 2°C/min, held for 0 seconds and lastly warmed to 40°C at a rate of 5°C/min.

Data was recorded as crystal count (y-axis) versus temperature (x-axis). At the beginning of temperature cycle, crystal count of the solution remained steady versus temperature defining a baseline crystal count value for the solution. When a sample froze upon cooling, the crystal count increased. During final warming cycle, when crystal count returned to original baseline, the temperature at that point was recorded as the Krafft point.

### Materials:

1 wt% solutions of each of the materials in the table below were tested. Solutions were prepared by mixing 1 wt% solutions of C12-NMG and C12-NMX materials in the indicated volumetric ratios. A volume of 0.150mL of this mixture was then analyzed via the Phase Technology® NK60-KPA Analyzer.

**Table 7. Krafft Measurements of C12-NMG:C12-NMX solutions**

| Solution | Percent 1 wt% C12-NMG (% by volume) | Percent 1 wt% C12-NMX (% by volume) | Krafft Point (°C) |
|---|---|---|---|
| 1 | 0 | 100 | 20 |
| 2 | 10 | 90 | 17 |
| 3 | 20 | 80 | 13 |
| 4 | 30 | 70 | 5-14** |
| 5 | 40 | 60 | 17 |
| 6 | 50 | 50 | 20 |
| 7 | 60 | 40 | 25 |
| 8 | 70 | 30 | 26 |
| 9 | 75 | 25 | 27 |
| 10 | 80 | 20 | 27 |
| 11 | 90 | 10 | 29 |
| 12 | 95 | 5 | 30 |
| 13 | 100 | 0 | 37 |

| | | | |
|---|---|---|---|
| **Significant drop in crystal count seen at 5°C but does not fully drop below line until 14°C | | | |

Mixtures of C12-NMG and C12-NMX amide have potential to result in lower Krafft points than the either of the pure materials with the optimal mixture being near 70% C12-NMX: 30% C12-NMG. When C12-NMG is mixed with materials based on a shorter starting sugar or polyol group such as C12-NMX, the physical thermal properties are improved beyond either C12-NMG or the C12-NMX. This improvement is shown with a drop in temperature of the Krafft point to a value below either C12-NMG or C12-NMX for the C12-NMG:C12-NMX system over a range of around 50 - 90% C12-NMX. Additionally, in systems where the majority of the material is C12-NMG, small additions (5% or more) of C12-NMX can lower the Krafft point of the mixture below that of C12-NMG alone.

### Example 34 - Synthesis of Coconut Oil N-Methylglucamide (CCO-NMG) using a Solvent:

A slurry of N-Methylglucamine (32.2g, 165 mmoles, Aldrich), RBD Coconut oil (35.2 g, 55 mmoles, AAK) and methanol (13.3 g, 20 wt% based on reactants) was heated to 80°C under N₂ blanket. Once at temperature, sodium methoxide (1.7 g of 25% in methanol, 8.0 mmoles, Aldrich) was added to reaction. Stirring for ∼30min at 80°C yielded a homogenous solution from which the excess methanol was removed by short-path distillation. Heating was continued for an additional 30 min at 80°C followed by 2 hrs at 90°C. After stirring at 90°C 2 hrs, the crude product was stirred an additional 1 hr under vacuum to a final pressure of 20 mm Hg yielding a tan paste, 68.1g, (92.9% desired products, 7.2% glycerin using GC using derivatization described in Example 1).

### Example 35 - Synthesis of Coconut Oil N-Methylxylamide (CCO-NMX) using a Solvent:

The procedure of Example 34 was used substituting N-Methylxylamine (40.8g of 65.8% solution in methanol, 165 mmoles) for N-Methylglucamide. After stirring at 90°C 2 hrs, the crude product was stirred an additional 1 hr under vacuum to a final pressure of 20 mm Hg yielding a tan paste (62.1 g, 91.0% desired products, 7% glycerin using GC using derivatization described in Example 1).

### Example 36 - Synthesis of Hydrogenated Coconut Oil, N-Methylglucamide (HCCO-NMG) without using a Solvent:

Hydrogenated Coconut oil (35.2 g, 55 mmoles, AAK) was melted at 80°C under N₂ blanket. To this was added 19.72g of N-Methylglucamine (NMG, yielding a viscous slurry) followed by sodium methoxide (1.7 g of 25% in methanol, 8.0 mmoles, Aldrich). Additional NMG (for a total of 32.2g, 165 mmoles, Aldrich) was added over 2.5 hours and the temperature was gradually increased to 110°C. During this time excess methanol was removed by short-path distillation. After stirring at 110°C 30 minutes, the crude product was stirred an additional 1 hr under vacuum to a final pressure of 20 mm Hg yielding a tan paste (67.2 g, 99% yield, 94.0% product/6% glycerin by GC using derivatization described in Example 1).

### Example 37 - CCO-NMG:CCO-NMX Mixed Sample Preparations

The procedure for Example 22 was followed using the title materials with vacuum drying run at ambient temperature instead of 50°C. Samples were then aged 10 days at 10°C

### Example 38 - Thermal Transition Determination via DSC of C12 CCO-NMG:CCO-NMX Mixed Samples

The procedure of Example 30 was followed using the mixed CCO-NMG:CCO-NMX samples described in Example 37. When placing the samples into the Tzero Hermetic pan & lid, it was noted that at ambient temperature, the samples containing 100% CCO-NMG and 75% CCO-NMG, 25% CCO-NMX, 50% CCO-NMG, 50% CCO-NMX, and 100% CCO-NMX were waxy in nature; and the sample containing 25% CCO-NMG, 75% CCO-NMX was a soft paste with the consistency of butter at room temperature.

**Table 8. Thermal Transition Determination of CCO-NMG:CCO-NMX by DSC**

| %CCO-NMG | %CCO-NMX | T Intercept (°C) | T Peak Max (°C) | T Intercept (°C) | T Peak Max (°C) |
|---|---|---|---|---|---|
| 100 | 0 | 49.6 | 66.3 | 118.4 | 119.5 |
| 75 | 25 | 39.3 | 58.8 | 123.5 | 124.1 |
| 50 | 50 | 26.0 | 42.7 | 121.4 | 124.9 |
| 25 | 75 | ** | ** | 116.7 | 118.5 |
| 0 | 100 | 21.7 | 34.6 | 86.2 | 90.3 |

| | | | | | |
|---|---|---|---|---|---|
| ** No peak was observed for this sample | | | | | |

Multiple phase transitions were noted for the samples shown above. The physical thermal properties of CCO-NMG and CCO-NMX as measured by DSC can be found in the data presented above. Interestingly enough, like Example 31 and 32 and unlike other examples, the initial low temperature transition was not observed for the mixture of 25% CCO-NMG and 75% CCO-NMX. The physical sample at this composition, however, was again a noticeable soft paste, as opposed to a wax as the other samples. The presence of a paste like consistency at 75% CCO-NMX, demonstrates improved thermal properties over the waxy solids of the other samples.

### Example 39 - Krafft Point Analysis of CCO-NMG:CCO-NMX Mixed Samples

The procedure of Example 33 was followed using the CCO-NMG and CCO-NMX samples described in Example 34 and 35, respectively, as noted below.

### Materials:

1 wt% solutions of each of the materials in the table below were tested. Solutions were prepared by mixing 1 wt% solutions of CCO-NMG and CCO-NMX materials in the indicated volumetric ratios. A volume of 0.150mL of this mixture was then analyzed via the Phase Technology® NK60-KPA Analyzer.

**Table 9. Krafft Measurements of CCO-NMG:CCO-NMX solutions**

| Percent 1 wt% CCO-NMG (% by volume) | Percent 1 wt% CCO-NMX (% by volume) | Krafft Point (°C) |
|---|---|---|
| 100 | 0 | 34 |
| 75 | 25 | 27 |
| 50 | 50 | 17 |
| 25 | 75 | 5 |
| 0 | 100 | 16 |

Mixtures of CCO-NMG and CCO-NMX have potential to result in lower Krafft points than the either of the pure materials with the optimal mixture being near 75% CCO-NMX: 25% CCO-NMG. When CCO-NMG is mixed with materials based on a shorter starting sugar or polyol group such as CCO-NMX, the physical thermal properties are improved beyond either CCO-NMG or the CCO-NMX. This improvement is shown with a drop in temperature of the Krafft point to a value of 5 °C, far below either 100% CCO-NMG at 34°C or 100% CCO-NMX at 16°C.

### Example 40 - Synthesis of C1214 N-Methylglucamide (C1214-NMG) Solution:

The procedure of Example 3 was used with N-Methylglucamine (582.6g, 3 moles, Aldrich), CE-1270, (70% Methyl-Dodecanoate, 30% Methyl-Tetradeconate, 669.2g, 3.15 moles, PGC) methanol (256.9g, Aldrich, anhydrous) and 25% sodium methoxide in methanol (84.3g, 0.39 moles, Aldrich). After stirring at 90°C 2 hrs, the crude product was stirred an additional 0.5 hr and stripped under partial vacuum. Anhydrous propylene glycol (110.1g, Aldrich, dried over 4A molecular sieves) was added and stripping was continued an additional 1hr to a final reaction weight of 1359g. This was poured into a tray and allowed to solidify overnight. GC analysis using derivatization procedure in Example 1 showed conversion of 97% active materials. Citric acid (25g, Aldrich) in ethanol (220g, Baker) was heated to 50°C and the product cake dissolved in this over 30 min. To this was added water (420g, HPLC grade, Baker) over 30 min yielding a final solutions composition of 58.7% active product, 5% propylene glycol, 10% ethanol, 30% water. pH = 6.78

### Example 41 - Synthesis of C1214 N-Methylxylamide (C1214-NMX) Solution:

Followed procedure in Example #40 using N-Methylxylamine (787.5g of 62.8% in methanol, 3 moles) in place of N-Methylglucamide with no additional methanol added to the reaction. After partially stripping, propylene glycol (108g) was added and stripping continued. 1284g crude product paste resulted which was allowed to stand overnight in the flask. GC analysis using derivatization procedure in Example 1 showed conversion of 96.5% active materials. Citric acid (25g), ethanol (216.4g) and water (366g) were used to prepare the final solutions (58.6% active product, 10% ethanol, 30% water. pH = 7.29)

### Example 42 - C1214-NMG:C1214-NMX Mixed Solution Preparations

500 g solutions of title material blends (ratio given by weight) were prepared by weighing appropriate amount of C1214-NMG solution from Example 40 in a beaker. Next the appropriate amount of C1214-NMX solution from Example 41 was added. Solutions of 70:30 C1214-NMG:C1214-NMX and 30:70 C1214-NMG:C1214-NMX were prepared in this way.

### Example 43 - Krafft Point Analysis of C1214-NMG:C1214-NMX Mixed Samples

The procedure of Example 33 was followed using the C1214-NMG, C1214-NMX and C1214-HMG:C1214-NMX solutions described in Example 40, 41 and 42 respectively, as noted below.

**Table 10. Krafft Measurements of C1214-NMG:C1214-NMX solutions**

| Sample Name | Krafft Point (°C) |
|---|---|
| C1214-NMG | 34 |
| 70:30 C1214-NMG:C1214-NMX | 28 |
| 30:70 C1214-NMG:C1214-NMX | 5 |
| C1214-NMX | 16 |

Mixtures of C1214-NMG and C1214-NMX solutions have potential to result in lower Krafft points than the either of the pure materials with a drastically lower point at 70% C1214-NMX and 30% C1214-NMG. When C1214-NMG is mixed with materials based on a shorter starting sugar or polyol group such as C1214-NMX, the physical thermal properties are improved beyond either C1214-NMG or the C1214-NMX. This improvement is shown with a drop in temperature of the Krafft point to a value of 5 °C, far below either 100% C1214-NMG at 34°C or 100% C1214-NMX at 16°C.

### Example 44 - Synthesis of N-Methyl Hexyl Glucamine (NMHG):

N-methylglucamine (25g, 128.6 mmol, Aldrich), Hexanal (15.5 g, 155 mmol, Aldrich), 5% Pd on Carbon (1 gram, Aldrich) and 40 mL of methanol were added to a 160 mL Parr Reactor. The reactor was sealed, purged three times with 300 PSI N₂ followed by three times with 300 PSI H₂. The reactor was then charged with 500 PSI H₂, at which point stirring was begun at 400 RPM, and was allowed to remain at room temperature for 1hr. The reactor was then externally heated to 50°C for 18 hrs, then 75°C for 1 hr, during which time pressure was maintained at 300-500 PSI H₂. The reactor was cooled to ambient temperature, vented and purged three times with 300 PSI N₂. The contents were filtered to remove the catalyst and the methanol removed to yield a white precipitant. Precipitant was filtered and washed with cold methanol to yield white powder,
(23 g, 98% desired products using GC using derivatization described in Example 1).

### Example 45 - Synthesis of N-Methyl Hexyl Xylamine (NMHX)

Followed procedure of Example 44 using n-methylxylamine (30.4g of 65 wt% solution in methanol, 120 mmol, synthesis described in Example 2) in place of n-methylglucamine and less hexanal (14.4 g, 144 mmol, Aldrich). (25.75 g, 95% desired products using GC using derivatization described in Example 1).

### Example 46 -NMHG:NMHX Mixed Sample Preparations

0.50g samples of title material blends (ratio given by weight) were prepared by weighing appropriate amount of individual materials and combining in glass vial with methanol (3 grams). The glass vials were stirred with magnetic stir bars and heated to 35°C for 1 hour. Solvent was allowed to evaporate under hood air flow and then aged 18 days at ambient temperature prior to thermal property analysis.

### Example 47 - Thermal Transition Determination via DSC of NMHG:NMHX Mixed Samples

The procedure of Example 30 was followed using the mixed NMHG:NMHX samples described in Example 46..

**Table 11. Thermal Transition Determination of NMHG:NMHX by DSC**

| % NMHG | %NMHX | T Intercept (°C) | T Peak Max (°C) |
|---|---|---|---|
| 100 | 0 | 73.8 | 79.0 |
| 75 | 25 | 70.0 | 73.8 |
| 50 | 50 | 46.7 | 56.0 |
| 25 | 75 | 40.2 | 51.1 |
| 0 | 100 | 48.6 | 52.3 |

The physical thermal properties of NMHG and NMHX along with mixtures of these two components as measured by DSC can be found in the data presented above. As with other examples, like Example 29, when NMHG is mixed with materials based on a shorter starting sugar or polyol group such as NMHX, the physical thermal properties are improved beyond either NMHG or NMHX. This improvement is shown with a drop in temperature of the initial phase transition (T Intercept in Table 11) to a value below either individual component for the NMHG:NMHX system over a range of around 50 - 90% NMHX.

### DISCUSSION

The physical thermal properties of pure sugar amide and amine systems can be found in the examples above. When materials based on glucose amides and amines such as C12-NMG are mixed with materials based on a shorter starting sugar or polyol group such as C12-NMX or C12-NMGly, the physical thermal properties are improved beyond either of the glucose derived amide/amine or the other sugar amide/amine with a shorter starting sugar or polyol group. This improvement in thermal properties has been measured by multiple techniques and been demonstrated for multiple thermal transitions including initial phase transition observed in DSC, initial visual melting points and Krafft Points. This improvement has further been demonstrated for multiple fatty chain lengths including both saturated and unsaturated examples. This improvement has been further demonstrated for mixtures of fatty chain lengths such as a mixed distribution from Coconut Oil, and has been demonstrated on mixed chains that are in solution, namely C1214 from CE-1270 (70% C12, 30% C14) at about 58% active in water, ethanol and propylene glycol. This improvement has lastly been shown for amines such as NMHG and NMHX.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A mixture comprising a first chemical and a second chemical, wherein said first chemical has the chemical structure of Formula I and said second chemical has the chemical structure of Formula II: 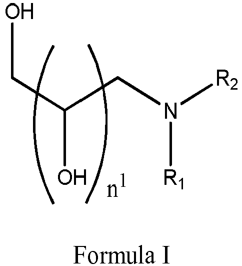 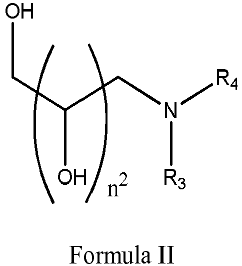 wherein:
n¹ is 2 to 4;
n² is 1 to 3;
n¹ is greater than n²
R₁ and R₃ are independently selected from C₁-C₁₆ alkyl, C₁-C₃ hydroxy- or methoxy-alkyl and;
R₂ and R₄ are independently selected from a structure of Formula III 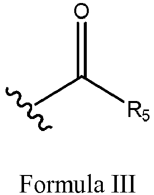
wherein: R₅ is C₇-C₂₃ alkyl, mono-alkenyl, di-alkenyl, tri-alkenyl, hydroxyl-alkyl, or hydroxyl-alkenyl, and mixtures thereof.

2. The mixture of claim 1, wherein n¹ is 4.

3. The mixture of claim 1, wherein a portion of the molecule of Formula I is derived from glucose.

4. The mixture of claim 1, wherein n² is 3.

5. The mixture of claim 1, wherein a portion of the molecule of Formula I is derived from glucose and a portion of the molecule of Formula II is derived from xylose.

6. The mixture of claim 5, wherein the weight ratio of the chemical structure of Formula I to the chemical structure of Formula II is from 50:50 to 0.5:99.5.

7. The mixture of claim 6, wherein R₁ and R₃ are methyl groups, and R₂ and R₄ have the structure of Formula III and R5 is a mixture of C11 and C13, wherein the weight ratio of C11 to C13 is between 99:1 and 60:40.

8. The mixture of claim 1, wherein the ratio of the first chemical to the second chemical is from 99.5:0.5 to 0.5:99.5.

9. The mixture of claim 1, wherein said mixture further comprises a third chemical, said third chemical having the chemical structure of Formula IV: 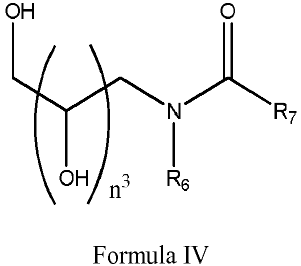 wherein:
R₆ is selected from hydrogen, C₁-C₁₆ alkyl, C₁-C₃ hydroxy- or methoxy-alkyl;
R₇ is independently selected from C₈-C₂₂ alkyl, mono-alkenyl, di-alkenyl, or tri-alkenyl and mixtures thereof;
n³ is 2 to 4.

10. The mixture of claim 9, wherein a portion of the molecule of Formula IV is selected from the group consisting of fragments derived from arabinose, galactose, mannose and combinations thereof.

11. A composition comprising
(a) from about 0.001 wt % to about 99.999 wt % of the mixture of claim 1, and
(b) from about 0.001 wt % to about 99.999 wt % of at least one additional component selected from the group consisting of cleaning components and personal care components.

12. A composition comprising
(a) from about 0.001 wt % to about 99.999 wt % of the mixture of claim 9, and
(b) from about 0.001 wt % to about 99.999 wt % of at least one additional component selected from the group consisting of cleaning components and personal care components.

13. The composition of claim 11 or 12, wherein at least one cleaning component is selected from the group consisting of a surfactant, a carrier, an enzyme, a builder, an alkalinity system, an organic polymeric compound, a hueing dye, a bleaching compound, an alkanolamine, a soil suspension agent, an anti-redeposition agent, a corrosion inhibitor, and mixtures thereof.

14. The composition of claim 11 or 12, wherein the composition is selected from the group consisting of a granular detergent, a bar-form detergent, a liquid laundry detergent, a liquid hand dishwashing mixture, a hard surface cleaner, a tablet, a disinfectant, an industrial cleaner, a highly compact liquid, a powder, a decontaminant, a shampoo, a hair conditioner, a hair treatment, a facial soap, a body wash, a body soap, a foam bath, a make-up remover, a skin care product, an acne control product, a deodorant, an antiperspirant, a shaving aid, a cosmetic, a depilatory, a fragrance, a lotion, and a mixtures thereof.

15. The composition of claim 11 or 12, wherein the personal care component is selected from the group consisting of an oil, and emollient, a moisturizer, a carrier, an extract, a vitamin, a mineral, an anti-aging compound, a surfactant, a solvent, a polymer, a preservative, an antimicrobial, a wax, a particle, a colorant, a dye, a fragrance, and mixtures thereof.

## Patentansprüche

1. Mischung, die eine erste Chemikalie und eine zweite Chemikalie umfasst, wobei die erste Chemikalie die chemische Struktur von Formel I aufweist und die zweite Chemikalie die chemische Struktur von Formel II aufweist: 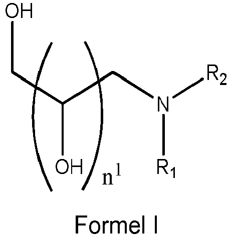 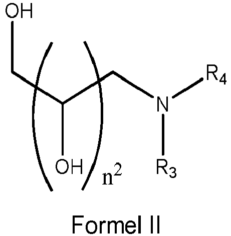 wobei:
n¹ 2 bis 4 beträgt;
n² 1 bis 3 beträgt;
n¹ größer als n² ist
R₁ und R₃ unabhängig voneinander ausgewählt sind aus C₁-C₁₆-Alkyl, C₁-C₃-Hydroxy- oder Methoxyalkyl, und;
R₂ und R₄ unabhängig voneinander ausgewählt sind aus einer Struktur von Formel III 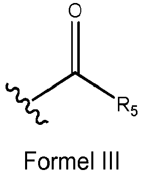 wobei: R₅ C₇-C₂₃-Alkyl, Monoalkenyl, Dialkenyl, Trialkenyl, Hydroxylalkyl, oder Hydroxylalkenyl, und Mischungen davon ist.

2. Mischung nach Anspruch 1, wobei n¹ 4 beträgt.

3. Mischung nach Anspruch 1, wobei ein Abschnitt des Moleküls von Formel I von Glukose abgeleitet ist.

4. Mischung nach Anspruch 1, wobei n² 3 beträgt.

5. Mischung nach Anspruch 1, wobei ein Abschnitt des Moleküls von Formel I von Glukose abgeleitet ist und ein Abschnitt des Moleküls von Formel II von Xylose abgeleitet ist.

6. Mischung nach Anspruch 5, wobei das Gewichtsverhältnis der chemischen Struktur von Formel I zur chemischen Struktur von Formel II von 50:50 bis 0,5:99,5 beträgt.

7. Mischung nach Anspruch 6, wobei R₁ und R₃ Methylgruppen sind und R₂ und R₄ die Struktur von Formel III aufweist und R5 eine Mischung aus C11 und C13 ist, wobei das Gewichtsverhältnis von C11 zu C13 zwischen 99:1 und 60:40 beträgt.

8. Mischung nach Anspruch 1, wobei das Verhältnis von erster Chemikalie zu zweiter Chemikalie von 99,5:0,5 bis 0,5:99,5. beträgt.

9. Mischung nach Anspruch 1, wobei die Mischung ferner eine dritte Chemikalie umfasst, wobei die dritte Chemikalie die chemische Struktur von Formel IV aufweist: 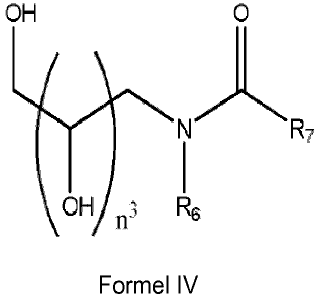 wobei:
R₆ ausgewählt ist aus Wasserstoff, C₁-C₁₆-Alkyl, C₁-C₃-Hydroxy- oder Methoxyalkyl;
R₇ unabhängig ausgewählt ist aus C₈-C₂₂-Alkyl, Monoalkenyl, Dialkenyl, oder Trialkenyl und Mischungen davon;
n³ 2 bis 4 beträgt.

10. Mischung nach Anspruch 9, wobei ein Teil des Moleküls von Formel IV ausgewählt ist aus der Gruppe bestehend aus Fragmenten, die von Arabinose, Galactose, Mannose und Kombinationen davon abgeleitet sind.

11. Zusammensetzung, umfassend:
(a) zu etwa 0,001 Gew.-% bis etwa 99,999 Gew.-% die Mischung nach Anspruch 1, und
(b) zu etwa 0,001 Gew.-% bis etwa 99,999 Gew.-% mindestens eine zusätzliche Komponente, die ausgewählt ist aus der Gruppe bestehend aus Reinigungskomponenten und Körperpflegekomponenten.

12. Zusammensetzung, umfassend:
(a) zu etwa 0,001 Gew.-% bis etwa 99,999 Gew.-% die Mischung nach Anspruch 9, und
(b) zu etwa 0,001 Gew.-% bis etwa 99,999 Gew.-% mindestens eine zusätzliche Komponente, die ausgewählt ist aus der Gruppe bestehend aus Reinigungskomponenten und Körperpflegekomponenten.

13. Zusammensetzung nach Anspruch 11 oder 12, wobei mindestens eine Reinigungskomponente ausgewählt ist aus der Gruppe bestehend aus einem Tensid, einem Träger, einem Enzym, einem Builder, einem Alkalinitätssystem, einer organischen polymeren Verbindung, einem Abtönungsfarbstoff, einer Bleichverbindung, einem Alkanolamin, einem Schmutzsuspendiermittel, einem Anti-Wiederablagerungsmittel, einem Korrosionsinhibitor, und Mischungen davon.

14. Zusammensetzung nach Anspruch 11 oder 12, wobei die Zusammensetzung ausgewählt ist aus der Gruppe bestehend aus einem granulären Reinigungsmittel, einem Reinigungsmittel in Stückform, einem flüssigen Wäschewaschmittel, einer flüssigen Handgeschirrspülmittelmischung, einem Reiniger für harte Oberflächen, einer Tablette, einem Desinfektionsmittel, einem Industriereiniger, einer hochkompakten Flüssigkeit, einem Pulver, einem Dekontaminationsmittel, einem Shampoo, einer Haarspülung, einer Haarbehandlung, einer Gesichtsseife, einem Körperwaschmittel, einer Körperseife, einem Schaumbad, einem Make-Up-Entferner, einem Hautpflegeprodukt, einem Akneregulierungsprodukt, einem Deodorant, einem Antitranspirant, einer Rasierhilfe, einem kosmetischen Mittel, einem Enthaarungsmittel, einem Duftstoff, einer Lotion, und einer Mischung davon.

15. Zusammensetzung nach Anspruch 11 oder 12, wobei die Körperpflegekomponente ausgewählt ist aus der Gruppe bestehend aus einem Öl, und Weichmacher, einem Feuchtigkeitsspender, einem Träger, einem Extrakt, einem Vitamin, einem Mineral, einem Alterungsverzögerer, einem Tensid, einem Lösungsmittel, einem Polymer, einem Konservierungsstoff, einem Konservierungsstoff, einem antimikrobiellen Mittel, einem Wachs, einem Partikel, einem Farbstoff, einem Färbemittel, einem Duftstoff, und Mischungen davon.

## Revendications

1. Mélange comprenant une première substance chimique et une deuxième substance chimique, dans lequel ladite première substance chimique a la structure chimique de la Formule I et ladite deuxième substance chimique a la structure chimique de la Formule II : 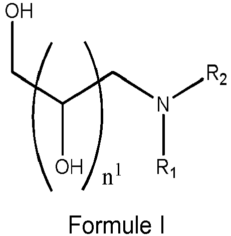 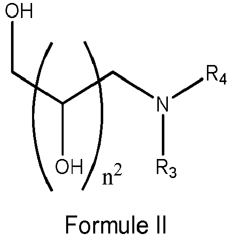 dans laquelle :
n¹ va de 2 à 4 ;
n² va de 1 à 3 ;
n¹ est supérieur à n²
R₁ et R₃ sont indépendamment choisis parmi un alkyle en C₁ à C₁₆, un hydroxy- ou méthoxy-alkyle en C₁ à C₃ et ;
R₂ et R₄ sont indépendamment choisis parmi une structure de Formule III 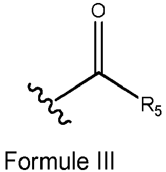 dans laquelle : R₅ est un alkyle, mono-alcényle, di-alcényle, tri-alcényle, hydroxyl-alkyle ou hydroxyl-alcényle, en C₇ à C₂₃, et des mélanges de ceux-ci.

2. Mélange selon la revendication 1, dans lequel n¹ vaut 4.

3. Mélange selon la revendication 1, dans lequel une partie de la molécule de Formule I est dérivée de glucose.

4. Mélange selon la revendication 1, dans lequel n² vaut 3.

5. Mélange selon la revendication 1, dans lequel une partie de la molécule de Formule I est dérivée de glucose et une partie de la molécule de Formule II est dérivée de xylose.

6. Mélange selon la revendication 5, dans lequel le rapport pondéral de la structure chimique de Formule I à la structure chimique de Formule II va de 50:50 à 0,5:99,5.

7. Mélange selon la revendication 6, dans lequel R₁ et R₃ sont des groupes méthyle, et R₂ et R₄ ont la structure de Formule III et R5 est un mélange de C11 et C13, dans lequel le rapport pondéral de C11 à C13 est compris entre 99:1 et 60:40.

8. Mélange selon la revendication 1, dans lequel le rapport de la première substance chimique à la deuxième substance chimique va de 99,5:0,5 à 0,5:99,5.

9. Mélange selon la revendication 1, où ledit mélange comprend en outre une troisième substance chimique, ladite troisième substance chimique possédant la structure chimique de la Formule IV : 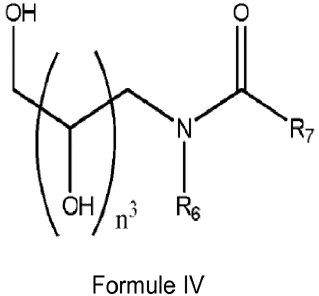 dans laquelle :
R₆ est choisi parmi l'hydrogène, un alkyle en C₁ à C₁₆, un hydroxy- ou méthoxy-alkyle en C₁ à C₃ ;
R₇ est indépendamment choisi parmi un alkyle, mono-alcényle, di-alcényle ou tri-alcényle en C₈ à C₂₂, et des mélanges de ceux-ci ;
n³va de 2 à 4.

10. Mélange selon la revendication 9, dans lequel une partie de la molécule de Formule IV est choisie dans le groupe constitué de fragments dérivés d'arabinose, de galactose, de mannose et des combinaisons de ceux-ci.

11. Composition comprenant
(a) d'environ 0,001 % en poids à environ 99,999 % en poids du mélange selon la revendication 1, et
(b) d'environ 0,001 % en poids à environ 99,999 % en poids d'au moins un composant supplémentaire choisi dans le groupe constitué de composants de nettoyage et composants de soin personnel.

12. Composition comprenant
(a) d'environ 0,001 % en poids à environ 99,999 % en poids du mélange selon la revendication 9, et
(b) d'environ 0,001 % en poids à environ 99,999 % en poids d'au moins un composant supplémentaire choisi dans le groupe constitué de composants de nettoyage et composants de soin personnel.

13. Composition selon la revendication 11 ou 12, dans laquelle au moins un composant de nettoyage est choisi dans le groupe constitué d'un agent tensioactif, un véhicule, une enzyme, un adjuvant, un système d'alcalinité, un composé polymère organique, une teinture teintante, un composé de blanchiment, une alcanolamine, un agent de suspension des salissures, un agent antiredéposition, un inhibiteur de corrosion, et des mélanges de ceux-ci.

14. Composition selon la revendication 11 ou 12, où la composition est choisie dans le groupe constitué d'un détergent granulaire, un détergent sous forme de pain, un détergent liquide pour le lavage du linge, un mélange liquide pour le lavage de la vaisselle à la main, un nettoyant pour surface dure, une tablette, un désinfectant, un nettoyant industriel, un liquide très compact, une poudre, un décontaminant, un shampooing, un conditionneur pour les cheveux, un traitement capillaire, un savon pour visage, un produit de lavage pour le corps, un savon pour le corps, un bain moussant, un démaquillant, un produit de soin de la peau, un produit de lutte contre l'acné, un déodorant, un anti-transpirant, une assistance au rasage, un produit de beauté, un dépilatoire, un parfum, une lotion, et des mélanges de ceux-ci.

15. Composition selon la revendication 11 ou 12, dans laquelle le composant de soin personnel est choisi dans le groupe constitué d'une huile, et un émollient, un agent hydratant, un véhicule, un extrait, une vitamine, un minéral, un composé anti-vieillissement, un agent tensioactif, un solvant, un polymère, un conservateur, un agent antimicrobien, une cire, une particule, un colorant, une teinture, un parfum et des mélanges de ceux-ci.
